# EUROPEAN PATENT APPLICATION

(11) **EP 2 039 325 A1**
(43) Date of publication of application: **25.03.2009**
(21) Application number: 08021269.9
(22) Date of filing: 02.05.2003
(51) Int. Cl.: A61F 2/24

(54) **Device for modifying the shape of a body organ**

(30) Priority: 08.05.2002 US 142637; 26.12.2002 US 331143
(62) Divisional of application: 03726639.2
(71) Applicant: Cardiac Dimensions, Inc., Kirkland, WA 98033 (US)
(72) Inventor: Mathis, Mark, L., Kirkland WA 98034 (US); Kowalsky, Leonard, Bothell WA 98021 (US); Reuter, David, G., Bothell WA 98021 (US); Beeson, Cruz, Kirkland WA 98034 (US); Nieminen, Gregory, D., Bothell WA 98021 (US); Braxtan, Ryan, H., Sammamish WA 98075 (US); Aronson, Nathan, Chico CA 959216 (US); Beget, Garrett, R., Bothell WA 98011 (US)
(74) Representative: Cole, William Gwyn

(57) **Abstract**

An intravascular support device includes a support or reshaper wire (102), a proximal anchor (140) and a distal anchor (120). The support wire engages a vessel wall to change the shape of tissue adjacent the vessel in which the intravascular support is placed. The anchors and support wire are designed such that the vessel in which the support is placed remains open and can be accessed by other devices if necessary.

## Description

### FIELD OF THE INVENTION

The present invention generally relates to a system and method to effect the shape of tissue structures within a body such as a mitral valve annulus of a heart The present invention more particularly relates to a mitral valve annulus device, system, and method wherein the device is deployed and anchored in the coronary sinus of a heart adjacent the mitral valve annulus to reshape the mitral valve annulus.

### BACKGROUND OF THE INVENTION

The human heart generally includes four valves. Of these valves, a most critical one is known as the mitral valve. The mitral valve is located in the left atrial ventricular opening between the left atrium and left ventricle. The mitral valve is intended to prevent regurgitation of blood from the left ventricle into the left atrium when the left ventricle contracts. In preventing blood regurgitation the mitral valve must be able to withstand considerable back pressure as the left ventricle contracts.

The valve cusps of the mitral valve are anchored to muscular wall of the heart by delicate but strong fibrous cords in order to support the cusps during left ventricular contraction. In a healthy mitral valve, the geometry of the mitral valve ensures that the cusps overlie each other to preclude regurgitation of the blood during left ventricular contraction.

The normal functioning of the mitral valve in preventing regurgitation can be impaired by dilated cardiomyopathy caused by disease or certain natural defects. For example, certain diseases may cause dilation of the mitral valve annulus. This can result in deformation of the mitral valve geometry to cause ineffective closure of the mitral valve during left ventricular contraction, Such ineffective closure results in leakage through the mitral valve and regurgitation. Diseases such as bacterial inflammations of the heart or heart failure can cause the aforementioned distortion or dilation of the mitral valve annulus. Needless to say, mitral valve regurgitation must not go uncorrected.

One method of repairing a mitral valve having impaired function is to completely replace the valve. This method has been found to be particularly suitable for replacing a mitral valve when one of the cusps has been severely damaged or deformed. While the replacement of the entire valve eliminates the immediate problem associated with a dilated mitral valve annulus, presently available prosthetic heart valves do not possess the same durability as natural heart valves.

Various other surgical procedures have been developed to correct the deformation of the mitral valve annulus and thus retain the intact natural heart valve function. These surgical techniques involve repairing the shape of the dilated or deformed valve annulus. Such techniques, generally known as annuloplasty, require surgically restricting the valve annulus to minimize dilation. Here, a prosthesis is typically sutured about the base of the valve leaflets to reshape the valve annulus and restrict the movement of the valve annulus during the opening and closing of the mitral valve.

Many different types of prostheses have been developed for use in such surgery. In general, prostheses are annular or partially annular shaped members which fit about the base of the valve annulus. The annular or partially annular shaped members may be formed from a rigid material, such as a metal, or from a flexible material.

While the prior art methods mentioned above have been able to achieve some success in treating mitral regurgitation, they have not been without problems and potential adverse consequences. For example, these procedures require open heart surgery. Such procedures are expensive, are extremely invasive requiring considerable recovery time, and pose the concomitant mortality risks associated with such procedures. Moreover, such open heart procedures are particularly stressful on patients with a compromised cardiac condition. Given these factors, such procedures are often reserved as a last resort and hence are employed late in the mitral regurgitation progression. Further, the effectiveness of such procedures is difficult to assess during the procedure and may not be known until a much later time. Hence, the ability to make adjustments to or changes in the prostheses to obtain optimum effectiveness is extremely limited. Later corrections, if made at all, require still another open heart surgery.

A new therapy to treat mitral regurgitation without resorting to open heart surgery has recently been proposed in U.S. Patent Nos. 6,210,432 and 6,402,781 as well as U.S. Patent Publication Nos. US 2001/0018611 A1, US 2001/0044568 A1, US 2002/0016628 A1, US 2002/0103533 A1, US 2002/0151961 A1, US 2002/0183835 A1, US 2002/0183836 A1, US 2002/0183837 A1, US 2002/0183838 A1, and US 2002/0183841 A1. This is rendered possible by the realization that the coronary sinus of a heart is near to and at least partially encircles the mitral valve annulus and then extends into a venous system including the great cardiac vein. As used herein, the term "coronary sinus" is meant to refer to not only the coronary sinus itself but in addition, the venous system associated with the coronary sinus including the great cardiac vein. The therapy contemplates the use of a device introduced into the coronary sinus to reshape and advantageously effect the geometry of the mitral valve annulus.

The device includes a resilient member having a cross sectional dimension for being received within the coronary sinus of the heart and a longitudinal dimension having an unstressed arched configuration when placed in the coronary sinus. The device partially encircles and exerts an inward pressure on the mitral valve. The inward pressure constricts the mitral valve annulus, or at least a portion of it, to essentially restore the mitral valve geometry. This promotes effective valve sealing action and eliminates mitral regurgitation.

The device may be implanted in the coronary sinus using only percutaneous techniques similar to the techniques used to implant cardiac leads such as pacemaker leads. One proposed system for implanting the device includes an elongated introducer configured for being releasably coupled to the device. The introducer is preferably flexible to permit it to advance the device into the heart and into the coronary sinus through the coronary sinus ostium. To promote guidance, an elongated sheath is first advanced into the coronary sinus. Then, the device and introducer are moved through a lumen of the sheath until the device is in position within the coronary sinus. Because the device is formed of resilient material, it conforms to the curvatures of the lumen as it is advanced through the sheath. The sheath is then partially retracted to permit the device to assume its unstressed arched configuration. Once the device is properly positioned, the introducer is then decoupled from the device and retracted through the sheath. The procedure is then completed by the retraction of the sheath. As a result, the device is left within the coronary sinus to exert the inward pressure on the mitral valve to restore mitral valve geometry.

The foregoing therapy has many advantages over the traditional open heart surgery approach. Since the device, system and method may be employed in a comparatively noninvasive procedure, mitral valve regurgitation may be treated at an early stage in the mitral regurgitation progression. Further, the device may be placed with relative ease by any minimally invasive cardiologist Still further, since the heart remains completely intact throughout the procedure, the effectiveness of the procedure may be readily determined. Moreover, should adjustments be deemed desirable, such adjustments may be made during the procedure and before the patient is sent to recovery.

While the technique of shoring up a mitral valve from within the coronary sinus appears promising, improvements may be made. The present invention is directed to improvements in implantable devices that are less likely to contribute to the formation of blockages in a vessel, are better anchored in vessel and are more easily delivered and placed in a vessel.

### SUMMARY OF THE INVENTION

The present invention is an intravascular support that is designed to change the shape of a body organ that is adjacent to a vessel in which the support is placed. In one embodiment of the invention, the support is designed to aid the closure of a mitral valve. The support is placed in a coronary sinus and vessel that are located adjacent the mitral valve and urges the vessel wall against the valve to aid its closure.

The intravascular support of the present invention includes a proximal and distal anchor and a support wire or reshaper disposed therebetween. The proximal and distal anchors circumferentially engage a vessel in which the support is placed, A support wire is urged against the vessel by the proximal and distal anchors to support the tissue adjacent the vessel.

In one embodiment of the invention, the proximal and distal supports are made from a wire hoop that presents a low metal coverage area to blood flowing within the vessel. The wire hoops may allow tissue to grow over the anchors to reduce the chance of thrombosis formation. The wire hoops have a figure eight configuration and can expand to maintain contact with the vessel walls if no vessel expands or changes shape.

In another embodiment of the invention, the proximal and distal anchors of the intravascular support are rotationally offset from each other. The anchor includes a fixation member carried on the device, the fixation member being adjustable from a first configuration that permits placement of the device in the body lumen to a second configuration that anchors the device within the body lumen, and a lock that locks the fixation member in the second configuration.

The lock is releasable to release the fixation member from the second configuration to permit the device to be removed from the body lumen. The fixation member may also be deformable to permit the device to be moved within the body lumen.

The fixation member is adjustable from the first configuration to a maximum second configuration. The lock may be configured to lock the fixation member at any one of a plurality of intermediate points between the first configuration and the maximum second configuration.

The fixation member may be elongated and have a first end hingedly coupled to the device body. The fixation member may thus extend along the device body closely spaced to the device body when in the first configuration and be pivoted from the device body to the second configuration to engage and anchor the device in the body lumen.

The anchor may further include a support that renders the fixation member substantially rigid when in the second configuration. The support may be an extension of the fixation member, wherein the fixation member includes a second end opposite the first end and wherein the lock locks the fixation member second end on the device body.

The fixation member may include a second end opposite the first end. The support may include a support member having a first end hingedly coupled to the fixation member second end and a second end opposite the support member first end. The lock may lock the support member second end on the device body. The support member second end may be slidable along the device body. The anchor may include a plurality of the fixation members and/or a plurality of support members.

The invention further provides a device that effects the condition of a mitral valve annulus of a heart. The device includes an elongated body dimensioned to be placed in the coronary sinus of the heart adjacent the mitral valve annulus. The device further includes a fixation member carried by the device, the fixation member being adjustable from a first configuration that permits placement of the device in the coronary sinus to a second configuration that anchors the device within the coronary sinus, and a lock that locks the fixation member in the second configuration.

The lock is releasable to release the fixation member from the second configuration to permit the device to be moved within the coronary sinus. The fixation member may be deformable to permit the device to be moved within the coronary sinus.

The fixation member may be adjustable from the first configuration to a maximum second configuration and the lock may lock the fixation member at any one of a plurality of intermediate points between the first configuration and the maximum second configuration.

The fixation member is elongated and has a first end hingedly coupled to the device body. The fixation member may extend along the device body closely spaced to the device body when in the first configuration and may be pivoted from the device body when in the second configuration to engage the coronary sinus and anchor the device in the coronary sinus. The device may further include a support that renders the fixation member substantially rigid when in the second configuration. The support may be an extension of the fixation member, wherein the fixation member includes a second end opposite the first end and wherein the lock locks the fixation member second end on the device body. The fixation member second end may be slidable along the device body and the device may include a plurality of the fixation members.

The fixation member may include a second end opposite the first end. The support may be a separate support member having a first end hingedly coupled to the fixation member second end and second end opposite the support member first end. The lock may then lock the support member second end on the device body. The support member second end may be slidable along the device body. The device may include a plurality of the fixation members and support members.

The invention further provides an assembly that effects the condition of a mitral valve annulus of a heart. The assembly includes a mitral valve therapy device dimensioned to be placed in the coronary sinus adjacent the mitral valve annulus. The device includes an elongated body, a fixation member carried by the device, the fixation member being adjustable from a first configuration that permits placement of the device in the coronary sinus to a second configuration that anchors the device within the coronary sinus, and a lock that locks the fixation member in the second configuration. The assembly further includes a flexible catheter having a lumen that receives the device and being dimensioned to be advanced into the coronary sinus to place the device adjacent the coronary sinus.

The assembly may further include an elongated pusher that is received by the lumen of the catheter proximal to the device and that permits the device and the catheter to be moved opposite each other. The assembly may further include a tether receivable by the catheter lumen and engagable with the device to pull the device distally with respect to the catheter. The catheter may be used to transition the fixation member from the first configuration to the second configuration. For example, the fixation member may be elongated and have a first end hingedly coupled to the device body. The fixation member may then extend along the device body when in the first configuration and the fixation member may be pivoted from the device body into the second configuration by distal movement of the catheter with respect to the device to cause the fixation member to engage the coronary sinus and anchor the device in the coronary sinus.

Another aspect of the present invention provides a mitral valve therapy device that reshapes the mitral valve annulus of the heart when placed within the coronary sinus of the heart adjacent the mitral valve annulus. The mitral valve therapy device has a proximal end including a coupling structure. The assembly further includes a catheter having a lumen that directs the mitral valve therapy device into the coronary sinus of the heart, a second coupling structure that is lockable on the device coupling structure, and a locking member that locks the device coupling structure to the second coupling structure and that releases the device coupling structure from the second coupling structure.

The assembly may further include a pusher member that pushes the device through the catheter lumen. The pusher member has a distal end that engages the device proximal end. The pusher member may carry the second coupling structure at the distal end of the pusher member.

The device coupling structure may comprise a hoop structure. The second coupling structure may also comprise a hoop structure. The locking member comprises a pin that extends through the hoop structures to lock the coupling structures together and that is retractable to release the hoop structures. The catheter has a distal end and the pin is preferably long enough to extend through the distal end of the catheter. The pusher member may be an elongated coil.

The device coupling structure and the second coupling structure may alternatively comprise a pair of interlocking structures and the locking member may comprise a slide-lock sheath closely fitted to the interlocking structures. The interlocking structures and the slide-lock sheath may be tubular. The pusher member has a distal end that engages the device proximal end, and carries the second coupling structure. The locking member may further include a tether that extends from the slide-lock sheath to and through the catheter lumen to permit the tether to pull proximally on the slide-lock sheath for releasing the interlocking structures.

The assembly may further include a retractor configured to extend through the catheter lumen and grip the device coupler. This permits retraction of the device through the catheter.

The invention further provides an assembly for effecting the condition of a mitral valve annulus of a heart comprising device means for reshaping the mitral valve annulus of the heart when placed within the coronary sinus of the heart adjacent the mitral valve annulus. The device means has a proximal end including a coupling means for coupling the device means. The assembly further comprises catheter means having a lumen that directs the mitral valve therapy device into the coronary sinus of the heart, second coupling means for locking with the device coupling means, and locking means for locking the device coupling means to the second coupling means and releasing the device coupling means from the second coupling means.

The present invention further provides a method of implanting a mitral valve therapy device to effect the condition of a mitral valve annulus of a heart. The method includes the steps of feeding a catheter having a lumen into the coronary sinus of the heart, locking the device to a deployment member with a locking member, and directing the mitral valve therapy device through the catheter lumen into the coronary sinus with the deployment member. The method further includes the steps of positioning the mitral valve therapy device in the coronary sinus with the deployment member, releasing the locking member from the device and the deployment member coupler, removing the deployment member and the locking member from the catheter lumen, and removing the catheter from the coronary sinus.

The invention still further provides a method of effecting the condition of a mitral valve annulus of a heart The method includes the steps of feeding a catheter having a lumen into the coronary sinus of the heart, aligning a mitral valve therapy device coupler of a mitral valve therapy device to a deployment member coupler, and locking the device coupler to the deployment member coupler with a locking member. The method further includes directing the mitral valve therapy device through the catheter lumen into the coronary sinus with the deployment member, positioning the mitral valve therapy device in the coronary sinus with the deployment member, releasing the locking member from the device coupler and the deployment member coupler, removing the deployment member, the deployment member coupler and the locking member from the catheter lumen, and removing the catheter from the coronary sinus.

The invention further provides an assembly for effecting the condition of a mitral valve annulus of a heart which includes a mitral valve therapy device that reshapes the mitral valve annulus of the heart when placed within the coronary sinus of the heart adjacent the mitral valve annulus, the mitral valve therapy device having a proximal end including a coupling structure and a guide member that directs the mitral valve therapy device into the coronary sinus of the heart. The assembly further includes a second coupling structure that is lockable on the device coupling structure, and a locking member that locks the device coupling structure to the second coupling structure and that releases the device coupling structure from the second coupling structure.

The invention further provides an assembly for effecting the condition of a mitral valve annulus of a heart. The assembly includes device means for reshaping the mitral valve annulus of the heart when placed within the coronary sinus of the heart adjacent the mitral valve annulus, the device means having a proximal end including a coupling means for coupling the device means, guide means for directing the mitral valve therapy device into the coronary sinus of the heart, second coupling means for locking with the device coupling means, and locking means for locking the device coupling means to the second coupling means and releasing the device coupling means from the second coupling means.

The invention still further provides a method of implanting a mitral valve therapy device to effect the condition of a mitral valve annulus of a heart. The method includes the steps of feeding a guide member into the coronary sinus of the heart, locking the device to a deployment member with a locking member, directing the mitral valve therapy device along the guide member into the coronary sinus with the deployment member, positioning the mitral valve therapy device in the coronary sinus with the deployment member, releasing the locking member from the device and the deployment member coupler, and removing the deployment member, the locking member, and the guide member from the coronary sinus.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 illustrates an intravascular support for changing the shape of an internal body organ in accordance with one embodiment of the present invention;
FIGURE 2 illustrates one method of deploying an intravascular support in accordance with the present invention;
FIGURE 3 illustrates one embodiment of the intravascular support in accordance with the present invention;
FIGURE 4 illustrates a distal anchor of the embodiment shown in FIGURE 3;
FIGURE 5 illustrates a proximal anchor of the embodiment shown in FIGURE 3;
FIGURES 6A-6C are cross-sectional views of crimp tubes for use with one embodiment of the present invention;
FIGURE 7 illustrates a proximal lock at the proximal end of the intravascular support as shown in FIGURE 3;
FIGURE 8 illustrates how the embodiment of the intravascular support shown in FIGURE 3 is deployed from a catheter;
FIGURE 9 illustrates an intravascular support in accordance with another embodiment of the present invention;
FIGURE 10 illustrates a distal anchor of the intravascular support shown in FIGURE 9;
FIGURE 11 illustrates a proximal anchor of the intravascular support shown in FIGURE 9;
FIGURE 12 illustrates yet another embodiment of an intravascular support in accordance with the present invention;

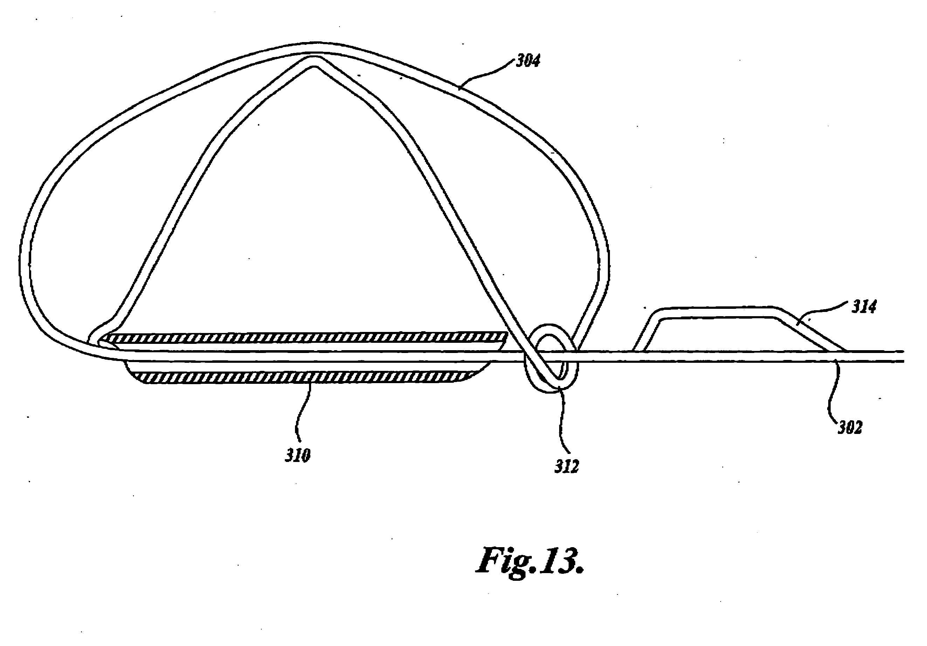

FIGURE 13 illustrates a distal anchor of the intravascular support shown in FIGURE 12;
FIGURE 14 illustrates a proximal anchor of the intravascular support shown in FIGURE 12;
FIGURE 15 illustrates an anchor and strut according to another embodiment of the invention;
FIGURE 16 illustrates a double loop anchor according to another embodiment of the invention;
FIGURE 17 illustrates a double loop anchor with a cross strut according to another embodiment of the invention;
FIGURE 18 illustrates an anchor with torsional springs according to another embodiment of the invention;
FIGURE 19 is a superior view of a human heart with the atria removed;
FIGURE 20 is a superior view of a human heart similar to FIGURE 19 illustrating a mitral valve therapy device including an anchor embodying the present invention deployed therein along with an assembly embodying the present invention for deploying the device;
FIGURE 21 is a side view with portions cut away illustrating a first step in deploying the device anchor of the device of FIGURE 20;
FIGURE 22 is a side view similar to FIGURE 21 illustrating a further step in the deployment of the anchor embodying the present invention;
FIGURE 23 is a side view similar to FIGURE 21 illustrating a further step in the deployment of the device anchor;
FIGURE 24 is a side view similar to FIGURE 21 illustrating the deployed device anchor;
FIGURE 25 is a side view similar to FIGURE 21 illustrating a first step in the removal of the device anchor,
FIGURE 26 is a side view similar to FIGURE 21 illustrating a final step in the removal of the device anchor;
FIGURE 27 is a side view similar to FIGURE 21 illustrating an alternate embodiment of a deployed device anchor embodying the present invention;
FIGURE 28 is a side view similar to FIGURE 21 illustrating a further embodiment of a deployed device anchor embodying the present invention;
FIGURE 29 is a side view similar to FIGURE 21 illustrating a still further embodiment of a deployed device anchor embodying the present invention;
FIGURE 30 is an end view of FIGURE 21;
FIGURE 31 is a superior view of a human heart with the atria removed;
FIGURE 32 is a superior view of a human heart similar to FIGURE 31 illustrating a mitral valve therapy device embodying the present invention deployed therein and which may be by deployed an assembly embodying the present invention;
FIGURE 33 is a superior view similar to FIGURE 31 with portions cut away illustrating the device of FIGURE 32 being deployed by a deployment assembly embodying the present invention;
FIGURE 34 is a partial perspective view to an enlarged scale illustrating the coupling members and locking member of a first embodiment of the present invention;
FIGURE 35 is a view similar to FIGURE 34 illustrating the release of the coupling structures;
FIGURE 36 is a superior view similar to FIGURE 31 illustrating recapture of the deployed device;
FIGURE 37 is a partial perspective view to an enlarged scale illustrating the recapture of the device;
FIGURE 38 is a superior view similar to FIGURE 31 illustrating a further embodiment of the present invention;
FIGURE 39 is a partial perspective view of the coupling and locking arrangement of FIGURE 38; and
FIGURE 40 is a partial perspective view illustrating the release of the coupling members of FIGURE 38.

### DETAILED DESCRIPTION OF THE PREFERRED EMBODIMENTS

As indicated above, the present invention is a medical device that supports or changes the shape of tissue that is adjacent a vessel in which the device is placed. The present invention can be used in any location in the body where the tissue needing support is located near a vessel in which the device can be deployed. The present invention is particularly useful in supporting a mitral valve in an area adjacent a coronary sinus and vessel. Therefore, although the embodiments of the invention described are designed to support a mitral valve, those skilled in the art will appreciate that the invention is not limited to use in supporting a mitral valve.

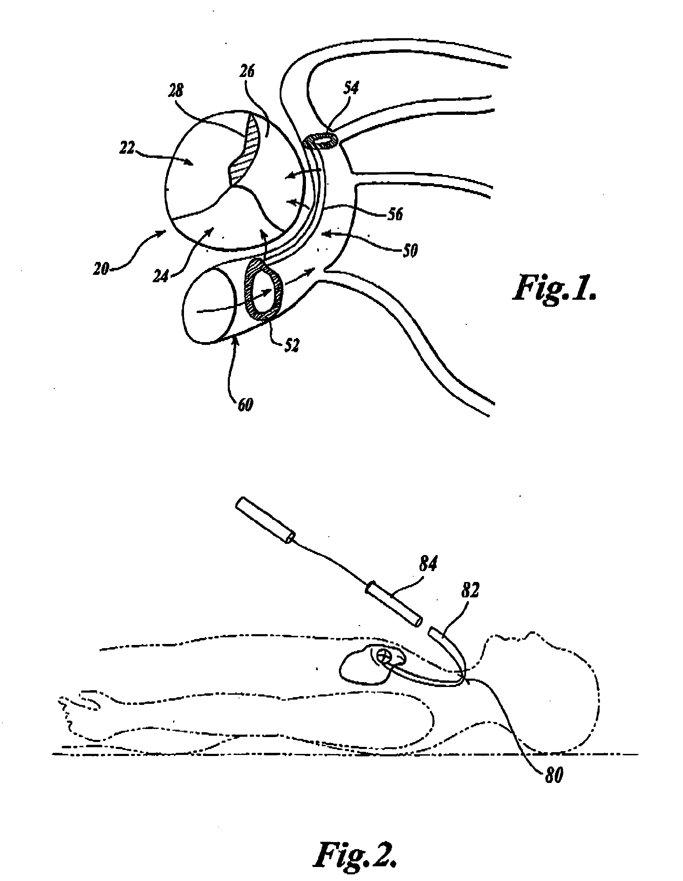

FIGURE 1 illustrates a mitral valve 20 having a number of flaps 22, 24 and 26 that should overlap and close when the ventricle of the heart contracts. As indicated above, some hearts may have a mitral valve that fails to close properly thereby creating one or more gaps 28 that allow blood to be pumped back into the left atrium each time the heart contracts. To add support to the mitral valve such that the valve completely closes, an intravascular support 50 is placed in a coronary sinus and vessel 60 that passes adjacent one side of the mitral valve 20. The intravascular support 50 has a proximal anchor 52, a distal anchor 54, and a support wire 56 or reshape extending between the proximal and distal anchors. With the anchors 52 and 54 in place, the support wire 56 exerts a force through the coronary sinus wall on the postero-lateral mitral valve 20 thereby closing the one or more gaps 28 formed between the valve flaps. With the intravascular support 50 in place, the function of the mitral valve is improved.

As will be explained in further detail below, each of the proximal and distal anchors 52, 54 preferably circumferentially engages the wall of the vessel 60 in which it is placed. The support wire 56 is secured to a peripheral edge of the proximal and distal anchors such that the support wite is urged by the anchors against the vessel wall. Therefore, the support wire 56 and anchors 52, 54 present a minimal obstruction to blood flowing within the vessel.

FIGURE 2 shows one possible method of delivering the intravascular support of the present invention to a desired location in a patient's body. An incision 80 is made in the patient's skin to access a blood vessel. A guide catheter 82 is advanced through the patient's vasculature until its distal end is positioned adjacent the desired location of the intravascular support. After positioning the guide catheter 82, a delivery catheter and advancing mechanism 84 are inserted through the guide catheter 82 to deploy the intravascular support at the desired location in the patient's body. Further detail regarding one suitable advancing mechanism 84 is described in commonly assigned U.S. Patent Application No. 10/313,914, filed December 5, 2002, the disclosure of which is hereby incorporated by reference.

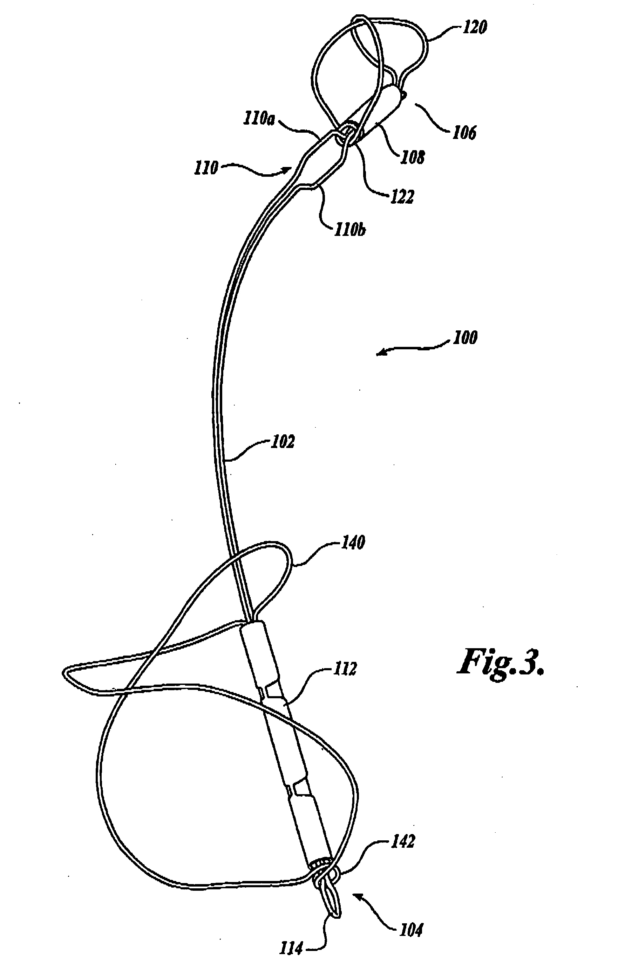

FIGURE 3 illustrates one embodiment of an intravascular support in accordance with the present invention. The intravascular support 100 includes a support wire 102 having a proximal end 104 and a distal end 106. The support wire 102 is made of a biocompatible material such as stainless steel or a shape memory material such as nitinol wire.

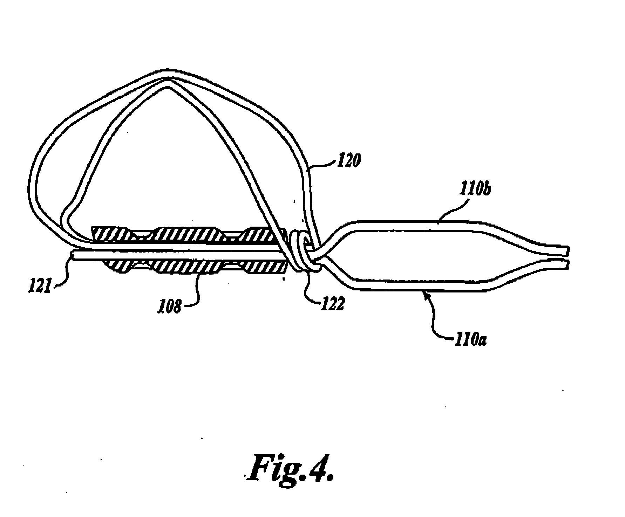

In one embodiment of the invention, the support wire 102 comprises a double length of nitinol wire that has both ends positioned within a distal crimp tube 108. To form the support wire 102, the wire extends distally from the crimp tube 108 where it is bent to form a distal stop loop (see 121 in FIGURE 4) having a diameter that is larger than the lumens within the distal crimp tube 108. After forming the distal stop loop, the wire returns proximally through the crimp tube 108 towards the proximal end of the support 100. Proximal to the proximal end of the crimp tube 108, is a distal lock 110 that is formed by the support wire bending away from the longitudinal axis of the support 102 and then being bent parallel to the longitudinal axis of the support before being bent again towards the longitudinal axis of the support. Therefore, the bends in the support wire form a half 110a of the distal lock that is used to secure the distal anchor in the manner described below. From the distal lock 110, the wire continues proximally through a proximal crimp tube 112. On exiting the proximal end of the proximal crimp tube 112, the wire is bent to form an arrowhead-shaped proximal lock 114. The wire of the support 102 then returns distally through the proximal crimp tube 112 to a position just proximal to the proximal end of the distal crimp tube 108 wherein the wire is bent to form a second half 110b of the distal lock 110.

Support wire 102 has a length that is selected based on its intended destination within a patient's vessel, For use in supporting a mitral valve, the support wire is preferably between one and six inches long and has a curved bend between its proximal end 104 and distal end 106 with a radius of curvature between 1 and 3 inches and most preferably with a radius of curvature of 1.8 inches. In addition, the wire used to form the support wire 102 is flexible enough to move with each heartbeat (thereby changing the force applied to the mitral valve annulus during the heartbeat) and stiff enough to support the mitral valve. In one embodiment, the wire used to form the support wire 102 is made of nitinol having a modulus of elasticity of 5-20 x 10⁶ psi and a diameter of between 0.0110" and 0.0150" and most preferably 0.0140". Other shape memory materials may be used for support wire as well.

At the distal end of the support wire 102 is a distal anchor 120 that is formed of a flexible wire such as nitinol or some other shape memory material. As is best shown in FIGURES 3 and 4, the wire forming the distal anchor has one end positioned within the distal crimp tube 108. After exiting the distal end of the crimp tube 108, the wire forms a figure eight configuration whereby it bends upward and radially outward from the longitudinal axis of the crimp tube 108. The wire then bends back proximally and crosses the longitudinal axis of the crimp tube 108 to form one leg of the figure eight. The wire is then bent to form a double loop eyelet or loop 122 around the longitudinal axis of the support wire 102 before extending radially outwards and distally back over the longitudinal axis of the crimp tube 108 to form the other leg of the figure eight Finally, the wire is bent proximally into the distal end of the crimp tube 108 to complete the distal anchor 120.

The distal anchor is expanded by sliding the double eyelet 122 of the distal anchor from a position that is proximal to the distal lock 110 on the support wire to a position that is distal to the distal lock 110. The bent-out portions 110a and 110b of support wire 110 are spaced wider than the width of double eyelet 122 and provide camming surfaces for the locking action. Distal movement of eyelet 122 pushes these camming surfaces inward to permit eyelet 122 to pass distally of the lock 110, then return to their original spacing to keep eyelet 122 in the locked position.

The dimensions of the distal anchor are selected so that the diameter of the distal anchor in a plane perpendicular to the axis of the lumen in which the anchor is deployed is preferably between 100% and 300%, most preferably between 130% and 200%, of the diameter of the lumen prior to deployment. When treating mitral valve regurgitation by placement of the device in the coronary sinus, the diameter of the coronary sinus may expand over time after deployment. Oversizing the anchor combined with the inherent deformability and recoverability properties of the anchor material (particularly nitinol or some other shape memory material) enables the anchor to continue to expand from its initial deployment size as the lumen distends and expands over time.

Upon expansion, the distal anchor circumferentially engages the vessel wall with a radially outwardly directed force that is distributed unequally around the circumference of the anchor by distending the vessel wall in variable amounts along the axial length of the anchor. The unequal distribution of force helps the anchor contact the lumen wall securely by creating bumps and ridges that are not parallel to the central axis of the lumen. In its expanded configuration the distal anchor's diameter is at least 50%-500% and most preferably 100%-300% of the anchor's diameter in the unexpanded configuration, The open cross-sectional area of the lumen through the anchor is at least 50% and most preferably 80%-100% of the lumen cross sectional area prior to redeployment of the anchor.

In addition, the metal coverage of the anchor, as defined by the percentage of the lumen surface area through which the anchor extends that is exposed to a metal surface, is between 5% and 30% and most preferably 10%. The wire used to form the distal anchor 120 is preferably Nitinol having a diameter of between 0.0110" and 0.0150" and most preferably 0.0140 inches. Other shape memory materials may be used as well.

During insertion, a physician can tactilely feel when the eyelet 122 has been slid over the distal lock 110 in order to determine when the distal anchor has been set within a vessel lumen. In addition, if the anchor is misplaced, it can be collapsed by pulling the eyelet 122 proximally over the distal lock 110 and repositioning the anchor in the unexpanded configuration. The force required to capture the distal anchor is preferably less than 20 lbs. and more preferably less than 10 lbs.

FIGURE 4 also illustrates how the crimp tube 108 is held in place between the distal lock 110 on the proximal side and the stop loop 121 at the distal end of the support wire 102. The wires of the distal anchor 120 exit the distal end of the crimp tube 108 at an angle of approximately 45 degrees before looping back over the length of the distal crimp tube 108. Therefore, the distal end of the anchor is relatively atraumatic to avoid damage to a vessel during placement

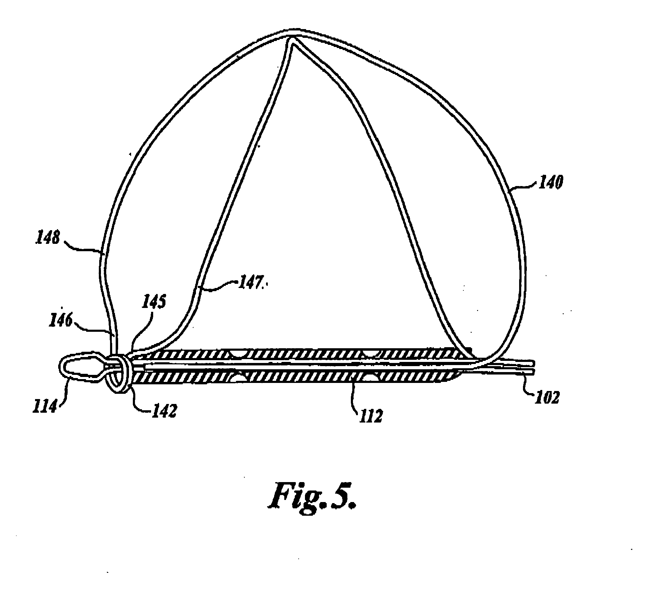

At the proximal end of the intravascular support is a proximal anchor 140 that is preferably formed of a biocompatible, elastic wire such as stainless steel or a shape memory material such as nitinol. As is best shown in FIGURES 3 and 5, the proximal anchor 140 in one embodiment is made of a single length of wire having a first end positioned within a proximal crimp tube 112. The wire extends distally from the crimp tube 112 and bends radially outward and away from the longitudinal axis of the crimp tube 112 before being bent proximally and crossing the longitudinal axis of the crimp tube 112 in order to form a first leg of a figure eight configuration. The wire then is bent to form a double eyelet or loop 142 around the longitudinal axis of the support wire 102 wherein the eyelet 142 has a diameter that allows it to be forced over the proximal lock 114. After forming the eyelet 142, the wire extends outwardly and away from the longitudinal axis of the crimp tube 112 before being bent dismally over and across the longitudinal axis of the crimp tube 112 to form the second leg of a figure eight. Finally, the wire is bent proximally and extends into the distal end of the crimp tube 112.

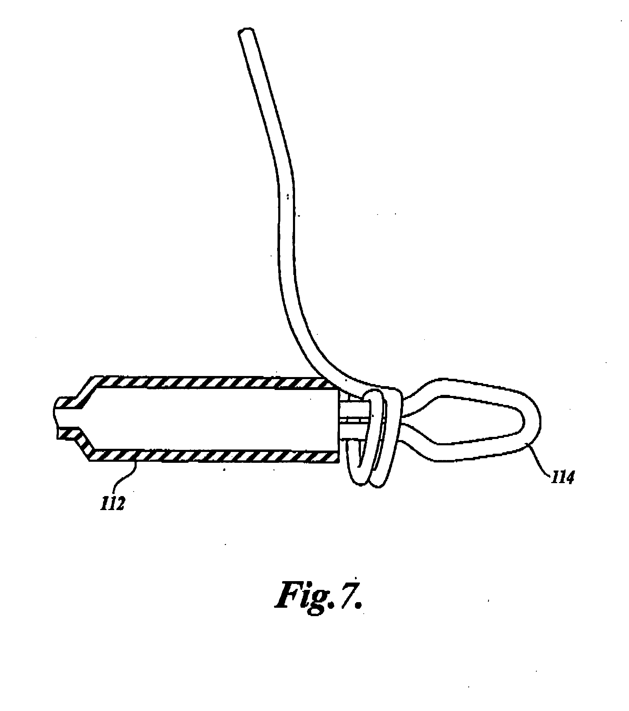

Like the distal anchor, the proximal anchor is expanded and locked by sliding the double eyelet 142 of the proximal anchor from a position that is proximal to the proximal lock 114 on the support wire to a position that is distal to the proximal lock 114. As can be seen in FIGURE 7, the proximal lock 114 has an "arrowhead" shape whereby the proximal end of the lock is bent away from the longitudinal axis of the support wire at an angle that is less steep than the distal end of the proximal lock. The less steep section makes it easier to advance the eyelet 142 over the lock in the distal direction than to retrieve the eyelet 142 over the proximal lock 114 in the proximal direction. Distal movement of eyelet 142 cams the less steep proximal surfaces inward to permit eyelet 142 to pass distally of the lock 114, then return to their original spacing to keep eyelet 142 in the locked position.

As can be seen by comparing the proximal anchor 140 with the distal anchor 120 in FIGURE 3, the proximal anchor has a larger radius of curvature because it is designed to fit within a larger diameter portion of the coronary sinus. The dimensions of the proximal anchor are selected so that the diameter of the proximal anchor in a plane perpendicular to the axis of the lumen in which the anchor is deployed is preferably between 100% and 300%, most preferably between 130% and 200%, of the diameter of the lumen prior to deployment. As with the distal anchor, oversizing the proximal anchor combined with the inherent deformability and recoverability properties of the anchor material (particularly nitinol or some other shape memory material) enables the anchor to continue to expand from its initial deployment size as the lumen distends and expands over time.

Upon expansion, the proximal anchor circumferentially engages the vessel wall with a radially outwardly directed an force that is distributed unequally around the circumference of the anchor by distending the vessel wall in variable amounts along the axial length of the anchor. As with the distal anchor, the unequal distribution of force helps the proximal anchor contact the lumen wall securely by creating bumps and ridges that are not parallel to the central axis of the lumen. In its expanded configuration the proximal anchor's diameter is at least 50%-500% and most preferably 100%-300% of the anchor's diameter in the unexpanded configuration. The open cross-sectional area of the lumen through the anchor is at least 50% and most preferably 80%-100% of the lumen cross sectional area prior to redeployment of the anchor.

In one embodiment of the invention, the proximal and distal anchors are oriented such that the planes of the anchors are offset with respect to each other by an angle of approximately 30 degrees. The offset helps the intravascular support 100 seat itself in the coronary sinus and vessel surrounding the mitral valve in certain mammals. However, it will be appreciated that if the support is designed for other uses, the proximal and distal anchors may be offset by more or less depending upon the anatomy of the intended destination.

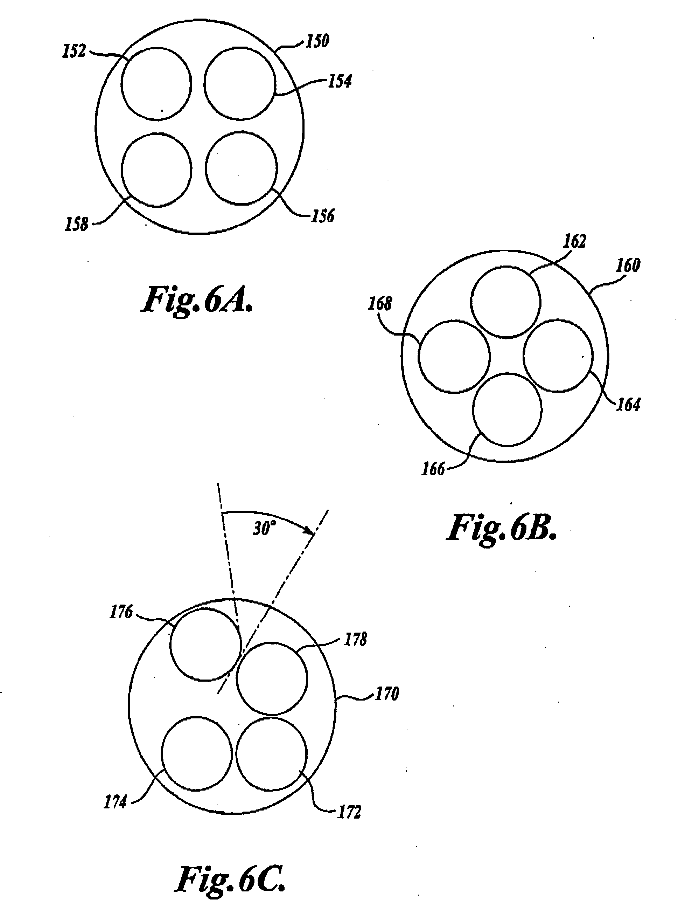

FIGURES 6A-6C illustrate cross-sectional views of the crimp tubes in which the wires that form the support wire 102 and proximal and distal anchors 120, 140 are threaded. In one embodiment, the crimp tubes comprise a biocompatible material such as titanium having a number of holes extending longitudinally through the tube through which the wires are threaded, In FIGURE 6A, a tube 150 has four holes 152, 154, 156, 158 positioned in approximately a square configuration within the circumference of the tube 150. As shown in FIGURE 6B, a tube 160 includes four holes 162, 164, 166, 168 therein that are positioned in a diamond configuration. FIGURE 6C shows another tube 170 having four holes 172, 174, 176, 178. Here the holes 172,174 lie in a first plane and the second pair of holes 176, 178 lie in a second plane that is offset from the plane of the holes 172,174. By changing the orientation of the holes 176, 178 with respect to the holes 172, 174, the relative plane of wires passing through the holes can be adjusted. Thus in the example shown in FIGURE 3, the proximal anchor may be formed with a crimp tube such as that shown in FIGURE 6A or FIGURE 6B while the proximal anchor may be formed in a crimp tube such as that shown in FIGURE 6C in order to adjust the angular orientation between the proximal anchor and the distal anchor. In an alternative embodiment, the crimp tubes at the proximal and distal ends of the support wire 102 are the same and the angular offset between the proximal and distal anchor is achieved by bending the wires at the desired angle. Although the crimp tubes shown use one hole for each wire passing through the crimp tube, it will be appreciated that other configurations may be provided such as slots or other passages for the wires to pass through.

In another embodiment, the distal and proximal anchors are attached to the support wire by a wire, such as nitinol wire or other shape memory material. The attaching wire may be spiral wrapped around the base of each anchor and around the support wire. In another embodiment, each anchor may be attached to the support wire by wrapping the anchor wire around the support wire. In yet another embodiment, the two anchors and the support wire may be made from a single wire, such as nitinol wire or other shape memory material.

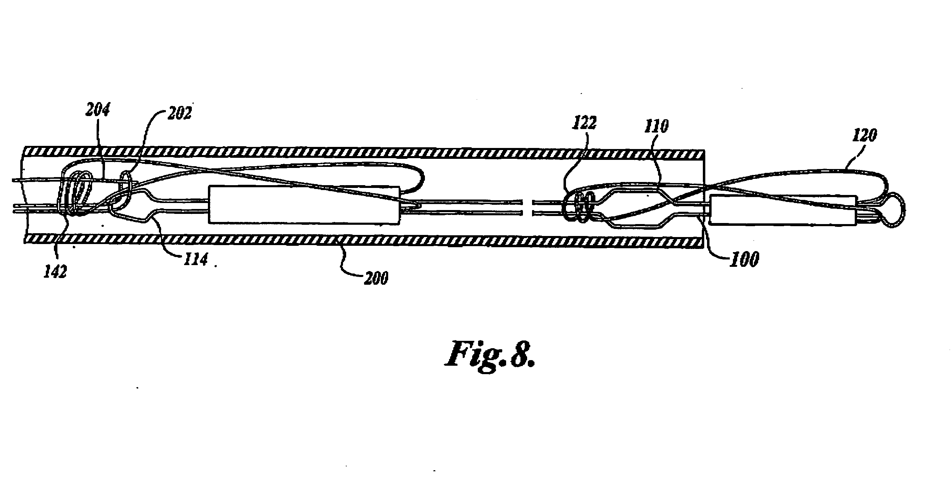

FIGURE 8 illustrates one method for delivering an intravascular support 100 in accordance with the present invention to a desired location in the body. As indicated above, intravascular support 100 is preferably loaded into and routed to a desired location within a catheter 200 with the proximal and distal anchors in a collapsed or deformed condition. That is, the eyelet 122 of the distal anchor 120 is positioned proximally of the distal lock 110 and the eyelet 142 of the proximal anchor 140 is positioned proximal to the proximal lock 114. The physician ejects the distal end of the intravascular support from the catheter 200 into the lumen by advancing the intravascular support or retracting the catheter or a combination thereof. A pusher (not shown) provides distal movement of the intravascular support with respect to catheter 200, and a tether 201 provides proximal movement of the intravascular support with respect to catheter 200. Because of the inherent recoverability of the material from which it is formed, the distal anchor begins to expand as soon as it is outside the catheter. Once the intravascular support is properly positioned, the eyelet 122 of the distal anchor is pushed distally over the distal lock 110 so that the distal anchor 120 further expands and locks in place to securely engage the lumen wall and remains in the expanded condition. Next, the proximal end of the support wire 102 is tensioned by applying a proximally-directed force on the support wire and distal anchor to apply sufficient pressure on the tissue adjacent the support wire to modify the shape of that tissue. In the case of the mitral valve, fluoroscopy, ultrasound or other imaging technology may be used to see when the support wire supplies sufficient pressure on the mitral valve to aid in its complete closure with each ventricular contraction without otherwise adversely affecting the patient. A preferred method of assessing efficacy and safety during a mitral valve procedure is disclosed in copending U.S. Patent Application Serial No. 10/366,585, filed February 12, 2003, and titled "Method of Implanting a Mitral Valve Therapy Device," the disclosure of which is incorporated herein by reference. Once the proper pressure of the support wire has been determined, the proximal anchor is deployed from the catheter and allowed to begin its expansion. The eyelet 142 of the proximal anchor 140 is advanced distally over the proximal lock 114 to expand and lock the proximal anchor, thereby securely engaging the lumen wall and maintaining the pressure of the support wire against the lumen wall. Finally, the mechanism for securing the proximal end of the intravascular support can be released. In one embodiment, the securement is made with a braided loop 202 at the end of tether 201 and a hitch pin 204. The hitch pin 204 is withdrawn thereby releasing the loop 202 so it can be pulled through the proximal lock 114 at the proximal end of the intravascular support 100.

In many contexts, it is important for the device to occupy as little of the lumen as possible. For example, when using the device and method of this invention to treat mitral valve regurgitation, the device should be as open as possible to blood flow in the coronary sinus (and to the introduction of other medical devices, such as pacing leads) while still providing the support necessary to reshape the mitral valve annulus through the coronary sinus wall. The combination of the device's open design and the use of nitinol or some other shape memory material enables the invention to meet these goals. When deployed in the coronary sinus or other lumen, the device preferably occupies between about 1.5% and about 5.5% of the overall volume of the section of lumen in which it is deployed.

In many embodiments of the invention, the use of a shape memory material such as nitinol is particularly important. The percentage of shape memory material by volume in the device is preferably between about 30% and 100%, most preferably between about 40% and 60%.

In some instances it may be necessary to move or remove an intravascular support after deployment by recapturing the device into a catheter. Prior to deployment of the proximal anchor, the distal anchor may be recaptured into the delivery catheter by simultaneously holding the device in place with tether 201 while advancing catheter distally over distal anchor 120 so that the entire device is once again inside catheter 200. The distally directed force of the catheter collapses distal anchor 120 into a size small enough to fit into catheter 200 again. Likewise, after deployment of both anchors but prior to releasing the securement mechanism as described above, the intravascular support may be recaptured into the delivery catheter by simultaneously holding the device in place with tether 201 while advancing catheter distally first over proximal anchor 140, over support wire 102, and finally over distal anchor 120. The distally directed forced of catheter 200 collapses anchors 120 and 140 into a size small enough to fit into catheter 200 again. If the securement mechanism has been detached from the device prior to recapture, the device still may be recaptured into the delivery catheter or another catheter by grasping the proximal end of the device with a grasper or tether and by advancing the catheter distally over the device.

In one embodiment of the invention, proximal anchor 140 includes a recapture guidance and compression element In the embodiment shown in FIGURE 5, the slope of the two proximal arms 143 and 144 of proximal anchor 140 is small in proximal portions 145 and 146 of the arms, then increases in more distal portions 147 and 148 of the arms. This shape guides the catheter to move distally over the anchor more easily and to help compress the anchor to a collapsed shape as the catheter advances during recapture.

Likewise, the two proximal arms 123 and 124 of distal anchor 120 have a shallower slope in their proximal portions 145 and 146 and an increased slope in more distal portions 147 and 148. While recapture of the distal anchor is somewhat easier due to its smaller size compared to the proximal anchor, this recapture guidance and compression feature enhances the ease with which recapture is performed.

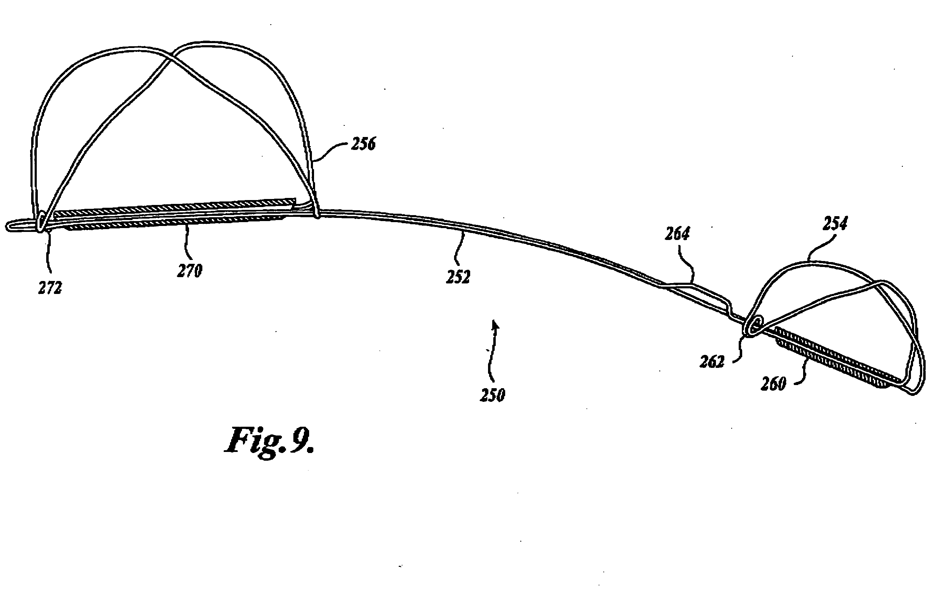

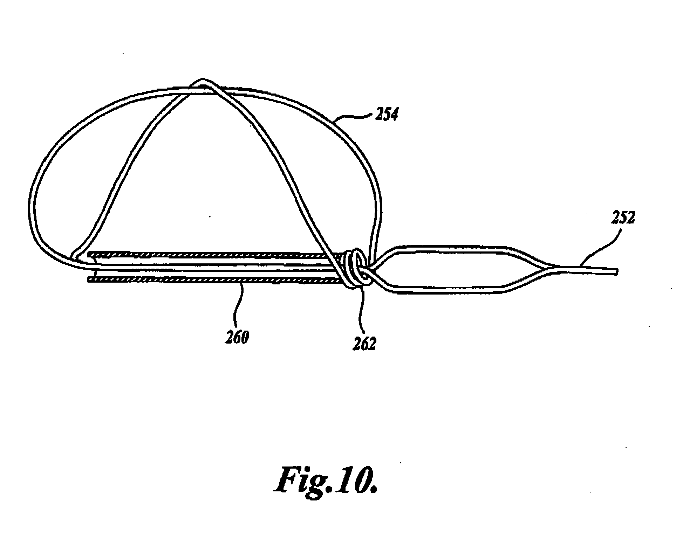

FIGURE 9 illustrates an alternative embodiment of the intravascular support of the present invention. In this embodiment, an intravascular support 250 has a support wire 252 and a distal anchor 254 and a proximal anchor 256. In the embodiment shown in FIGURE 9, the distal anchor 254 is made from the same wire used to form the support wire 252. As best shown in FIGURE 10, the wire used to form the support wire 252 extends distally through a distal crimp tube 260 before looping radially outward and returning proximally and across the longitudinal axis of the crimp tube 260 to form one leg of a figure eight. The wire then winds around the axis of the suspension wire 252 to form an eyelet 262. The wire then continues radially outward and distally across the longitudinal axis of the crimp tube 260 to form the second leg of a figure eight. After forming the figure eight, the wire enters the distal end of the crimp tube 260 in the proximal direction to form the other half of the support wire 252. A distal lock 264 is formed proximal to the distal crimp tube 260 by outwardly extending bends in the wires that form the support wire 252. The distal lock 264 prevents the double eyelet 262 from sliding proximally and collapsing the distal anchor 254 when positioned in a vessel.

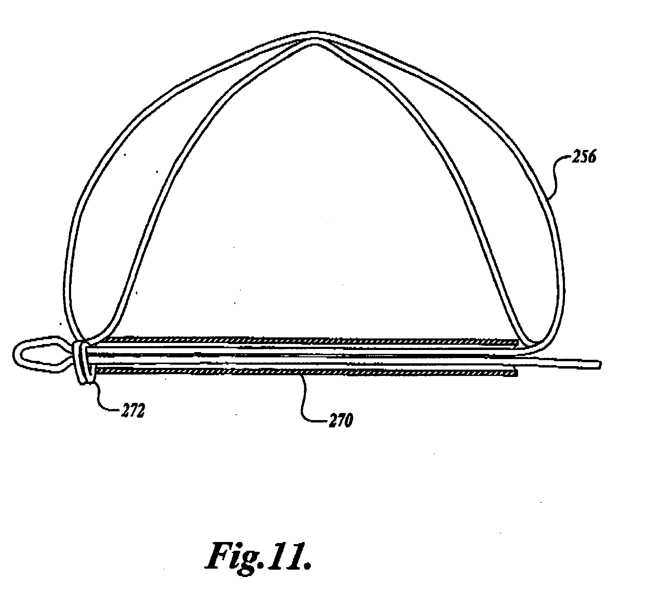

As shown in FIGURE 11, a distal anchor 256 is constructed in a fashion similar to the proximal anchor 140 shown in FIGURE 3. That is, the proximal anchor 256 is formed of a separate wire than the wire used to form the support wire 252 and distal anchor 254. The wire of the proximal anchor has one end within a proximal crimp tube 270. The wire extends distally out of the end of the crimp tube and bends radially outward before returning back and across the longitudinal axis of the crimp tube 270. At the proximal end of the crimp tube 270, the wire of the proximal anchor forms a double eyelet 272 around the longitudinal axis of the support wire 252. The wire then continues radially outward and distally over the longitudinal axis of the crimp tube 270 to form the second leg of the figure eight whereupon it is bent proximally into the distal end of the crimp tube 270.

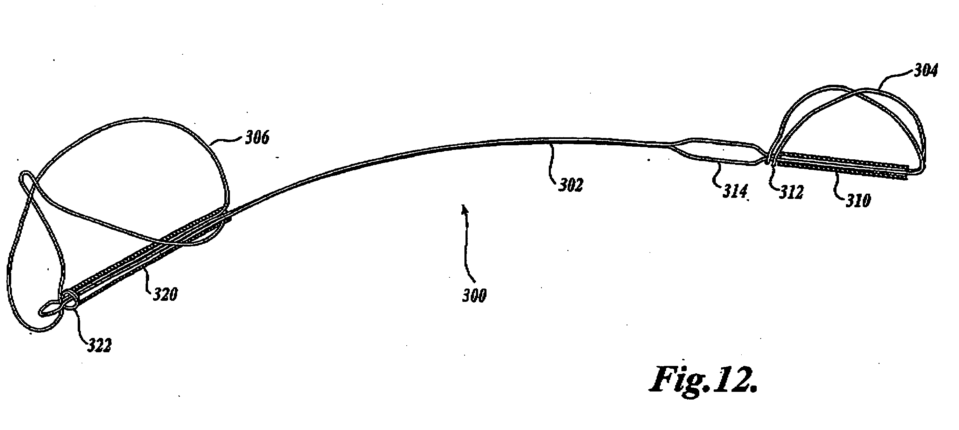

FIGURE 12 shows yet another embodiment of an intravascular support in accordance with the present invention. Here, an intravascular support 300 comprises a support wire 302, a distal anchor 304 and a proximal anchor 306. As in the embodiment shown in FIGURE 9, the distal anchor 304 and the support wire 302 are formed of the same wire. To form the distal anchor, the wire extends distally through a distal crimp tube 310 and exits out the distal end before extending radially outward and bending back and across the longitudinal axis of the crimp tube 310 to form one leg of a figure eight. The loop then forms an eyelet 312 around the longitudinal axis of the support wire 302 before bending radially outward and distally across the longitudinal axis of the crimp tube 310 to form a second leg of the figure eight. The wire then enters the distal end of the crimp tube 310 in the proximal direction. The support wire 302 may have one or two outwardly extending sections that form a distal stop 314 to maintain the position of the eyelet 312 once the distal anchor is set in the expanded configuration.

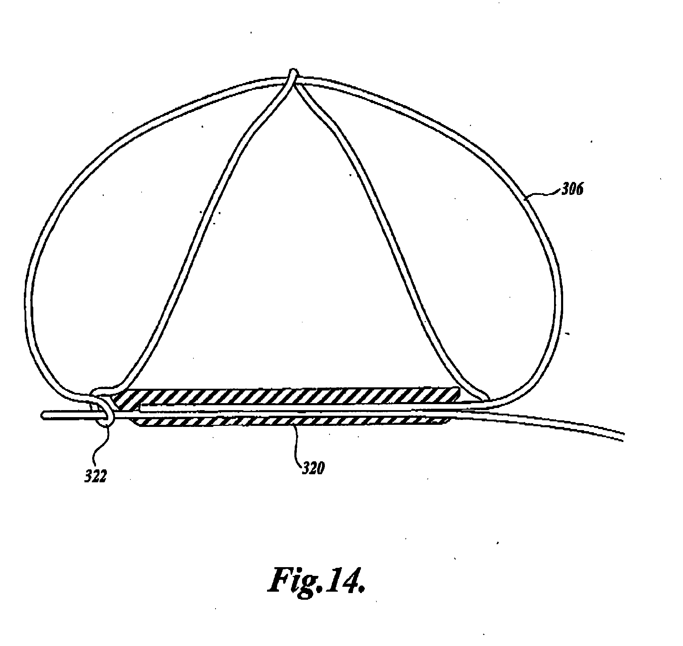

The proximal anchor 306 is formed from a separate wire as shown in FIGURE 14. The wire has one end positioned within the proximal crimp tube 320 that extends distally outward and radially away from the longitudinal axis of the crimp tube 320 before being bent proximally and across the longitudinal axis of the crimp tube 320 to form one leg of the figure eight. The wire then winds around the longitudinal axis of the support wire to form an eyelet 322 before being bent distally and across the longitudinal axis of the crimp tube 320 to enter the distal end of the crimp tube 320 in the proximal direction. As will be appreciated, the proximal crimp tube 320 of the embodiment shown in FIGURE 12 holds four wires wherein the distal crimp tube 310 need only hold two wires.

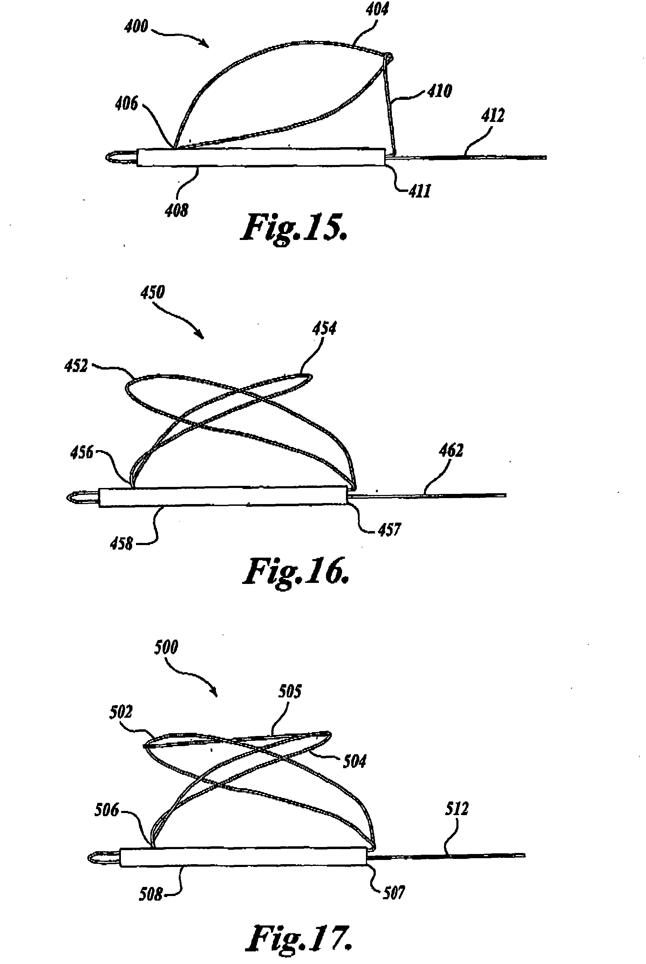

FIGURES 15-18 show other embodiments of the invention. In the embodiment shown in FIGURE 15, the intravascular support has an anchor 400 formed as a loop 404 emerging from a window 406 in a crimp tube 408. Extending from one end 411 of crimp tube 408 is a support strut 410 which connects with loop 404, Also extending from the crimp tube 408 is a support wire 412. Loop 404 and support 410 may be formed from nitinol, stainless steel, or any other appropriate material. The intravascular support includes another anchor. The intravascular support of this embodiment may be delivered and deployed in the manner discussed above with respect to the embodiment described above.

FIGURE 16 shows another embodiment of an anchor 450 for an intravascular support. Anchor 450 is formed from two loops 452 and 454 emerging from a window 456 and an end 457 of a crimp tube 458. A support wire 462 also extends from the crimp tube. Loops 452 and 454 may be formed from nitinol, stainless steel, or any other appropriate material. The intravascular support includes another anchor. The intravascular support of this embodiment may be delivered and deployed in the manner discussed above with respect to the embodiment described above.

FIGURE 17 shows yet another embodiment of an anchor 500 for an intravascular support according to this invention. Anchor 500 is formed from two loops 502 and 504 emerging from a window 506 and an end 507 of a crimp tube 508. A cross strut 505 connects the loops. A support wire 512 also extends from the crimp tube. Loops 502 and 504 and strut 505 may be formed from nitinol, stainless steel, or any other appropriate material. The intravascular support includes another anchor. The intravascular support of this embodiment may be delivered and deployed in the manner discussed above with respect to the embodiment described above.

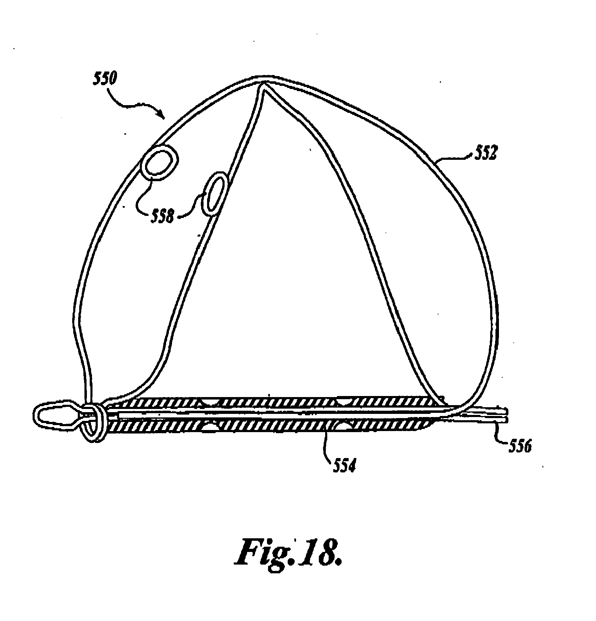

FIGURE 18 is a modification of the embodiment shown in FIGURES 3-7. In this embodiment, torsional springs 558 of proximal anchor 550 have been formed as single loops or eyelets in the anchor's wire 552. These springs make the anchor 550 more compliant by absorbing some of the force applied to the anchor during locking. While FIGURE 18 shows a proximal anchor with two springs 558, any number of springs could be used on either the proximal or the distal anchor.

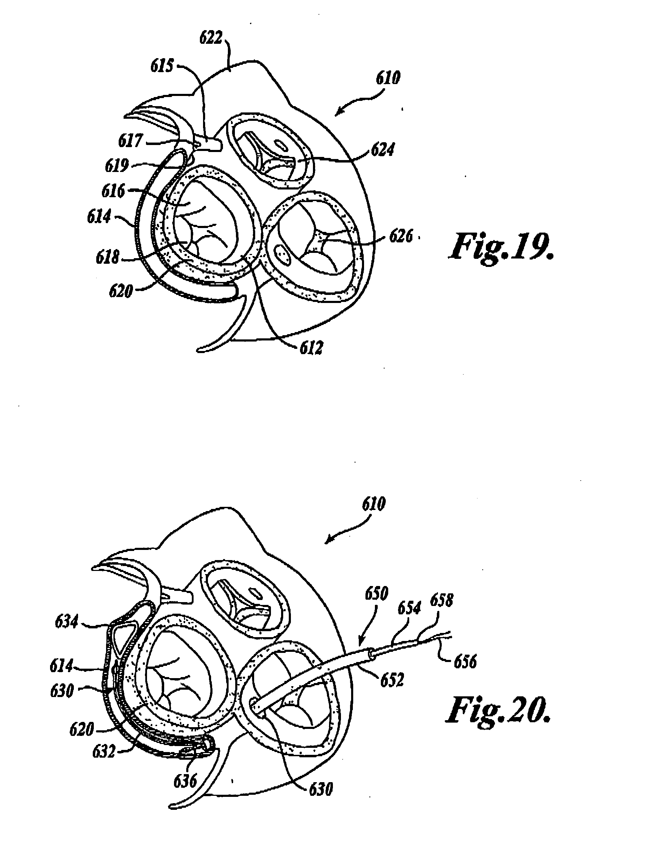

Referring now to FIGURE 19, it is a superior view of a human heart 610 with the atria removed to expose the mitral valve 612, the coronary sinus 614, the coronary artery 615, and the circumflex artery 617 of the heart 610 to lend a better understanding of the present invention. Also generally shown in FIGURE 19 are the pulmomary valve 622, the aortic valve 624, and the tricuspid valve 626 of the heart 610,

The mitral valve 612 includes an anterior cusp 616, a posterior cusp 618 and an annulus 620. The annulus encircles the cusps 616 and 618 and maintains their spacing to provide a complete closure during a left ventricular contraction. As is well known, the coronary sinus 614 partially encircles the mitral valve 612 adjacent to the mitral valve annulus 620. As is also known, the coronary sinus is part of the venous system of the heart and extends along the AV groove between the left atrium and the left ventricle. This places the coronary sinus essentially within the same plane as the mitral valve annulus making the coronary sinus available for placement of the mitral valve therapy device of the present invention therein.

FIGURE 20 shows a mitral valve therapy device 630 embodying the present invention shown deployed in the coronary sinus 614 of the heart 610 adjacent the mitral valve annulus 620 for effecting the geometry of the mitral valve annulus. Also shown in FIGURE 20 is a deployment system 650 that deploys the device 630 in the coronary sinus 614. The device 630 takes the form of an elongated body 632 which includes a distal anchor 634 embodying the present invention and a proximal anchor 636.

The anchors 634 and 636 are shown in FIGURE 20 in their deployed configuration. As will be seen hereinafter, upon deployment of the device 630 in the coronary sinus, the distal anchor 634 is transitioned from a first configuration to a locked second configuration. In the process, it is expanded outwardly to anchor the device in the coronary sinus against both bi-directional longitudinal and rotational movement. The proximal anchor however, when deployed, is configured to permit proximal movement This allows the device 630 to be tightened within the coronary sinus by proximal pulling of the anchor 636 after the distal anchor 634 is deployed. The device 630 may be formed from Nitinol or stainless steel, for example.

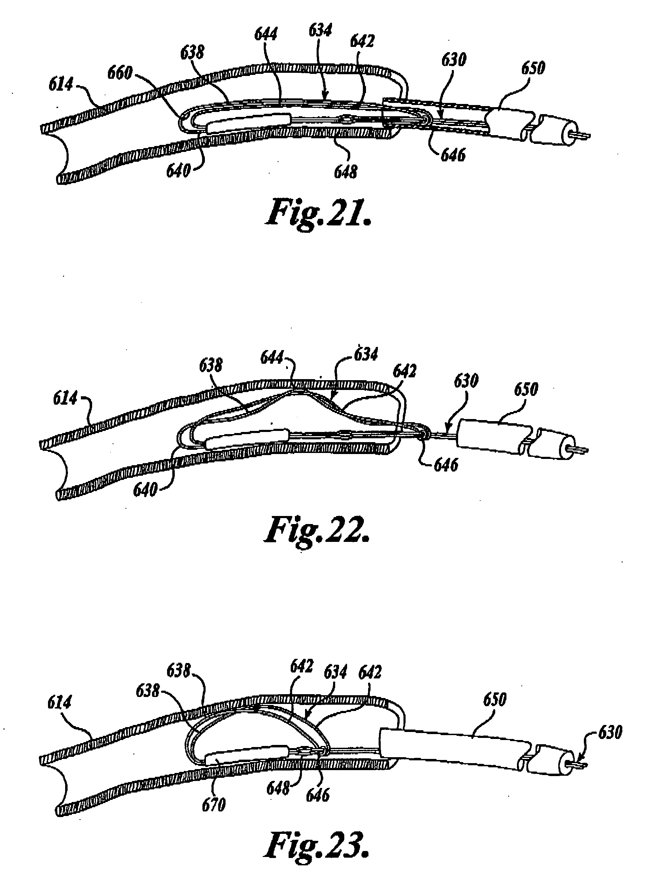

The deployment system 650 illustrated in FIGURE 20 includes an elongated catheter 652, an elongated pusher 654, and a tether 656. In deploying the device 630, the tether 656 is first looped about the proximal anchor 636 of the device 630 as illustrated and the device is then loaded into the catheter 650. The tether 656 is then threaded through an internal lumen 658 of the pusher 654 and looped around the proximal anchor 636 of the device 630 as illustrated. The pusher 654 is then advanced along the tether 656 for engaging the device 630 and pushing the device distally down the catheter to a predetermined position at the distal end of the catheter 650. The catheter with the device 630 loaded therein is then fed into the heart and through the coronary sinus ostium 630 into the coronary sinus to place the catheter in a position such that the device 630 is adjacent the mitral valve annulus 620. Thereafter, the device is maintained in a stationary position by the pusher 654 as the catheter 650 is partially withdrawn to expose the distal anchor 634. Once the distal anchor is exposed, it is deployed by the catheter in a manner to be described more particularly with respect to FIGURES 21-24. Once the distal anchor 634 is deployed, the catheter 650 is then retracted proximally of the proximal anchor 636. This exposes the proximal anchor 636 and permits the proximal anchor to self deploy. Once the proximal anchor is deployed, the tether 656 is pulled proximally to move the proximal anchor 636 in a proximal direction for tightening the device within the coronary sinus and to an extent which results in the desired effect on the geometry of the mitral valve annulus 620. During this adjustment process, mitral regurgitation may be monitored and the device adjusted for optimal results. When the device 630 is in its final position within the coronary sinus 614, the pusher 654 and catheter 650 may be removed from the heart. The tether 656 may be permitted to remain in the heart during an acute phase to ascertain the effectiveness of the device 630. Should further adjustment of the device be necessary, the tether 656 may then be used as a guide for guiding the introduction of the catheter 650 back into the heart.

FIGURES 21-24 illustrate the manner in which the distal anchor 634 may be deployed in the coronary sinus 614 for anchoring the device 630. It will be appreciated by those skilled in the art, of course, that the anchor 634 may be utilized in body lumens other than the coronary sinus and with therapeutic devices other than the mitral valve annulus therapy device illustrated in FIGURE 20.

In each of FIGURES 21-24 a portion of the coronary sinus has been removed and the pusher has not been illustrated so as to not unduly complicate the figures. FIGURE 21 shows the catheter 650 disposed within the coronary sinus 614 with the device 630 and distal anchor within the catheter 650. To that end, the catheter includes a lumen 660 which is dimensioned to receive the device 630 and the distal anchor 634 when the distal anchor 634 is in a first configuration. The distal anchor 634 includes an elongated fixation member 638 which is hingedly coupled to the distal end of the device 630 at a hinge 640. The elongated fixation member thus extends along the body of the device 630. The fixation member includes a support 642 which is an extension of the fixation member 638 and which is hingedly connected to the fixation member 638 at a hinge point 644. The proximal end of the fixation member 638 includes a loop 646 which is looped about the device 630 to permit the loop 646 to slide along the device 630. As will be seen subsequently, the loop 646 forms part of a lock for locking the anchor 634 in a second configuration for anchoring in the coronary sinus.

To complete the anchor, the device 630 includes a resilient enlarged portion 648 over which the loop 646 may slide. Once the loop 646 is located distally of the enlarged portion 648, it will be held by the enlarged portion 648 for locking the device in the second configuration.

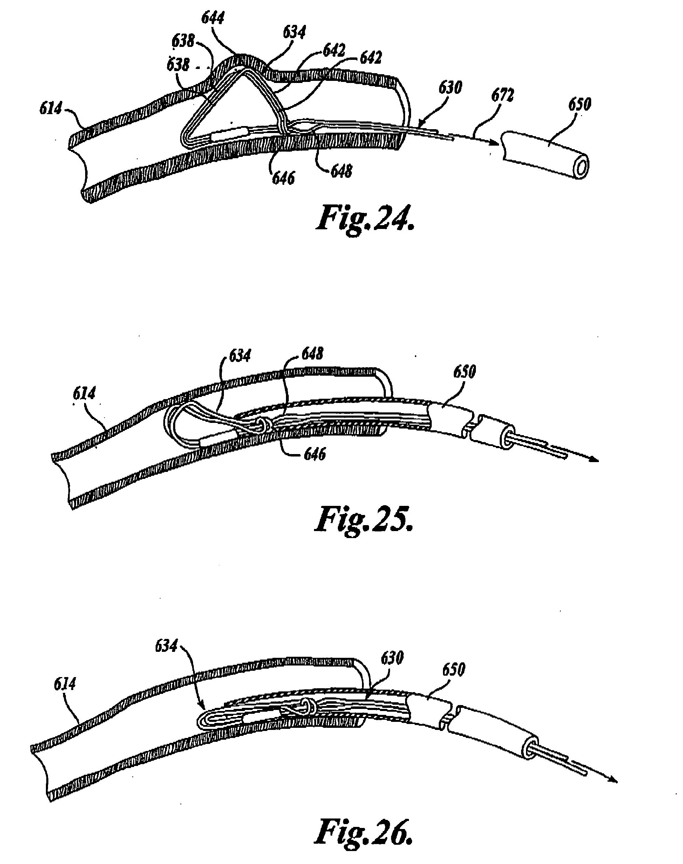

FIGURE 22 illustrates the anchor 634 after the catheter 650 has been moved proximal to the anchor 634. More specifically, it will be noted that the distal end of the catheter 650 is now proximal to the loop 646 or proximal end of the anchor 634. The shape memory of the anchor has caused the anchor to expand and is now partially transitioned from the first configuration of FIGURE 21 to the second and final configuration to be described with reference to FIGURE 24 subsequently.

FIGURE 23 illustrates the anchor 634 being transitioned from the first configuration to the second configuration. This transition is implemented by the distal end of the catheter 650 pushing the proximal end of the anchor 634 in the distal direction. To maintain the position of the anchor 634 during the transition, the tether 656 is used to hold the device 630 against distal movement.

The particular configuration of the distal anchor 634 in accordance with this embodiment may be more particularly seen in FIGURE 23. Here it may be seen that the distal anchor is formed of a wire having a first end secured to the distal end of the device 630, folded back and looped around the device and then back to the distal end of the device. Both ends of the anchor are then crimped by a crimp 670. This configuration results in a pair of fixation members 638 each having a support extension 642. In addition, the fixation members 638 may be formed so as to have a loop configuration to maximize surface contact with the inner wall of the coronary sinus 614.

As the catheter 650 is moved distally, it forces the loop 646 of the anchor 634 over the enlarged portion 648 of the device 630 to a point distal to the enlarged portion 648. This locks the loop 646 distally of the enlarged portion 648 for locking the anchor 634 in an enlarged second configuration as illustrated in FIGURE 24 to anchor the device 630 within the coronary sinus 614. More specifically, it may be seen that the supports 642 have been pivoted at the hinge 644 relative to the fixation member 638. This allows the fixation members 638 to be supported by the supports 642 and securely locked by the lock of the loop 646 and enlarged portion 648 of the device 630. The fixation members 638 provide broad surface contact with the inner wall of the coronary sinus 614. This provides for anchoring within the coronary sinus of the device 630 against both bi-directional longitudinal and rotational movement. Once the anchor 634 is deployed as illustrated in FIGURE 24, the catheter 650 may then be removed as indicated by the arrow 672.

One of the many features of the anchor of the instant invention is that it may be moved within or removed from the body lumen in which it is deployed. More specifically, and making reference to FIGURE 24, the anchor 634 may be removed by grabbing the support members 642 and pulling the loop 646 over the resilient enlarged portion 648 of the device 630. When the loop 646 is on the proximal side of the enlarged portion 648, further proximal movement of the loop 646 will fully transition the anchor 634 from the second configuration back to the first configuration for removal within the catheter 650.

Alternatively, by virtue of the support members, the anchor 634 may be formed of deformable material such as stainless steel. Using this to advantage, the anchor 634 may be partially collapsed by the catheter 650 to permit the anchor 634 and hence the device 630 to be moved and repositioned in the coronary sinus after which the resilience of the anchor material returns the anchor to its locked and deployed configuration. The anchor may be collapsed by the catheter 650 as illustrated in FIGURES 25 and 26.

In FIGURE 25, it will be noted that the catheter 650, while the device is held stationary by the tether, is moved distally over the enlarged portion 648 and the loop 646. The anchor 634 is now partially collapsed for movement and repositioning. Once repositioned, the catheter may be withdrawn to redeploy the anchor 634 which returns to its second configuration by virtue of its resiliency and shape memory.

As seen in FIGURE 26, continued distal movement of the catheter 650 causes the anchor 634 to fully collapse. This allows the anchor 634 to be totally drawn into the catheter 650. Once the anchor 634 is collapsed and within the catheter 650, the device 630 may be removed by removing the catheter with the device therein or by pulling the device proximally through the catheter.

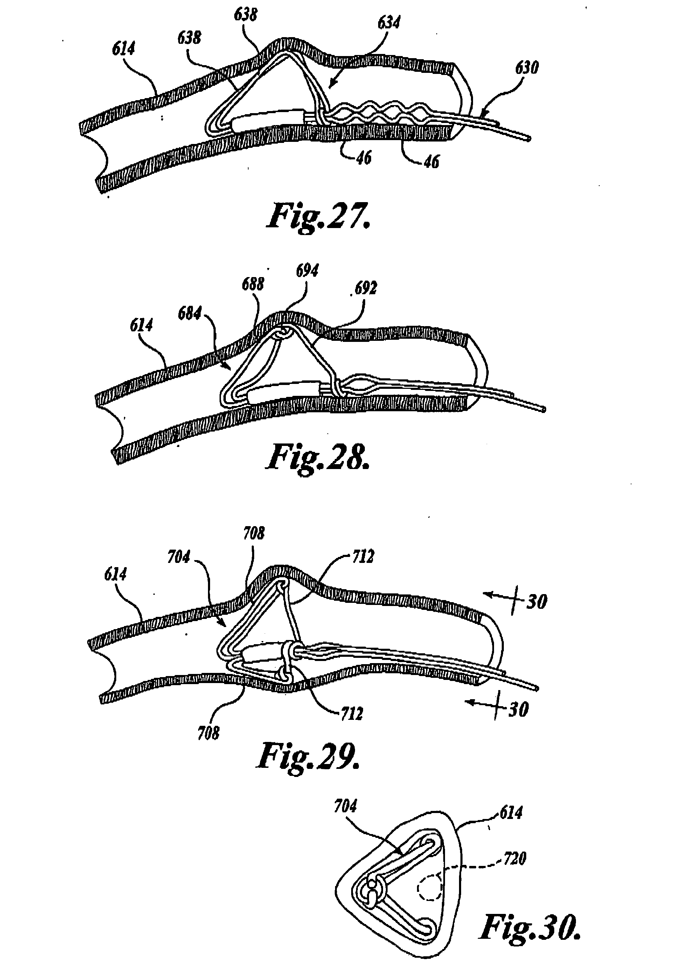

FIGURES 27-30 illustrate alternative embodiments of the anchor of the present invention. These embodiments are once again illustrated in connection with the anchoring of a mitral valve annulus therapy device within the coronary sinus of a heart.

In FIGURE 27, the device 630 is shown having a plurality of enlarged portions 646. As a result, a plurality of locks are provided on the device 630 to enable the fixation members to be locked at any one of a plurality of intermediate points between the first configuration and a maximum second configuration illustrated in FIGURE 27. This enables the anchor 634 to be sized to a given body lumen.

FIGURE 28 shows another anchor 684 embodying the present invention which has a separate fixation member 688 and support member 692. The second or distal end of the fixation member 688 is hingedly coupled to a first or distal end of the support member 692 by a hinged connection 694. The fixation member 688 may have a hoop configuration as the fixation members 638 previously described.

FIGURES 29 and 30 illustrated a still further anchor 704 having a pair of fixation members 708 and corresponding separate support members 712. Here, the fixation members 708 are formed by immediately adjacent anchor wires which, as best seen in FIGURE 30, are disposed at an angle to permit a cardiac lead, indicated by the dashed circle 720, to pass through the anchor and thus be within the coronary sinus. Hence, a device having an anchor such as anchor 704 is compatible with the provision of a cardiac lead therewith.

As can thus been seen, the present invention provides a new and improved anchor for anchoring a therapeutic device within a body lumen. The anchor of the present invention, by virtue of the lockable support member, creates mechanical advantage to assist deployment of the anchor. This also increases anchor strength. Because the support members may be of hooped or looped configuration, increased contact area between the anchor and the body lumen can be achieved. In addition, the anchor of the present invention allows deactivation and repositioning of the anchor or therapeutic device incorporating the anchor. Still further, because of the locked support structure, the anchor may be formed of smaller diameter wire, tube wall, or other materials which without the locked support provided by the anchor of the present invention would be unsuitable for this application.

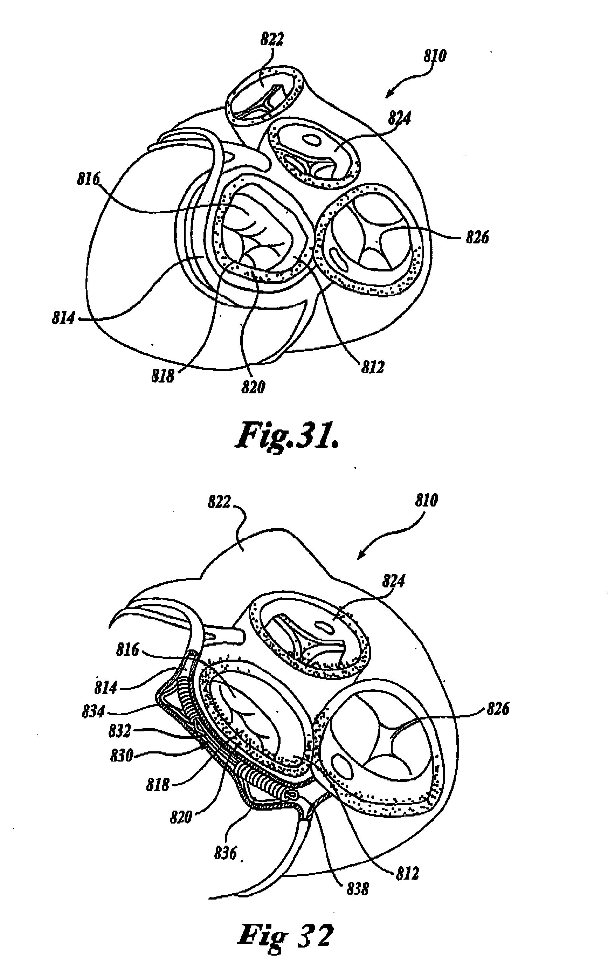

Referring now to FIGURE 31, it is a superior view of a human heart 810 with the atria removed to expose the mitral valve 812, and the coronary sinus 814 of the heart 810. Also generally shown in FIGURE 31 are the pulmonary valve 822, the aortic valve 824, and the tricuspid valve 826 of the heart 810.

The mitral valve 812 includes an anterior cusp 816, a posterior cusp 818 and an annulus 820. The annulus encircles the cusps 816 and 818 and maintains their spacing to provide a complete closure during a left ventricular contraction. As is well known, the coronary sinus 814 partially encircles the mitral valve 812 adjacent to the mitral valve annulus 820. As is also known, the coronary sinus is part of the venus system of the heart and extends along the AV groove between the left atrium and the left ventricle. This places the coronary sinus essentially within the same plane as the mitral valve annulus making the coronary sinus available for placement of the mitral valve therapy device of the present invention therein.

FIGURE 32 shows a mitral valve therapy device 830 embodying the present invention shown deployed in the coronary sinus 814 of the heart 810 adjacent the mitral valve annulus 820 for effecting the geometry of the mitral valve annulus. The device 830 takes the form of an elongated body 832 which includes a distal anchor 834 and a proximal anchor 836.

The anchors 834 and 836 are shown in FIGURE 32 in their deployed configuration. In deploying the device 830 in the coronary sinus, the distal anchor 834 is first deployed to anchor the distal end of the device 830. In the anchoring process, the anchor 834 is expanded outwardly to anchor the device in the coronary sinus against both bi-directional longitudinal and rotational movement This allows the device 830 to be tightened within the coronary sinus by pulling of the device's proximal end. Then, the proximal anchor 836 is deployed. The device 830, which may be formed from Nitinol or stainless steel, for example, now exerts an inward pressure on the mitral valve annulus 820 to advantageously effect its geometry.

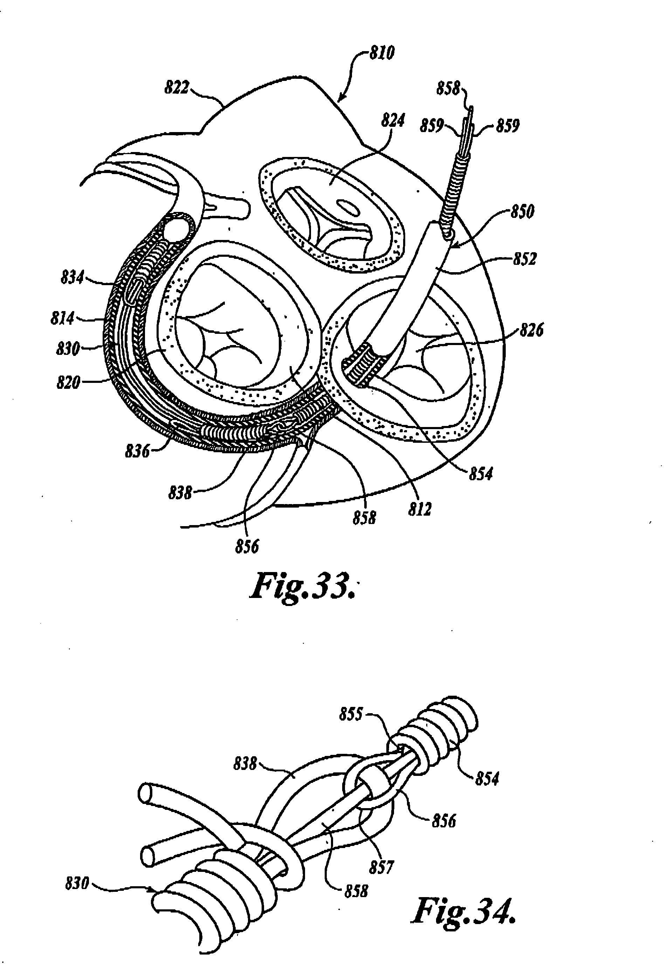

The device 830 along with its deployment system 850 is illustrated in FIGURE 33. As shown, the device is in the process of being implanted in the coronary sinus 814 of the heart 810. Its proximal anchor 836 and distal anchor 834 have yet been deployed. The deployment system 850 includes an elongated catheter 852, an elongated pusher 854, a coupling structural member 856 and a locking pin 858. As may be noted in FIGURE 34, the proximal end of the device 830 includes a coupling loop 838. The pusher 854 is preferably an elongated coil having a center lumen 855. The coupling member 856 is formed from a cable that is provided with a loop 857. The legs or ends 859 of the loop 857 extend proximally through the lumen 855 and out the proximal end of the pusher 854.

The locking pin 858 also extends proximally out of the proximal end of the pusher 854. As shown in FIGURE 34, the coupling loops 838 and 857 are aligned to overlap and the locking pin 858 is extended through the overlapping loops. This causes the device 830 to be releasably locked to the pusher 854.

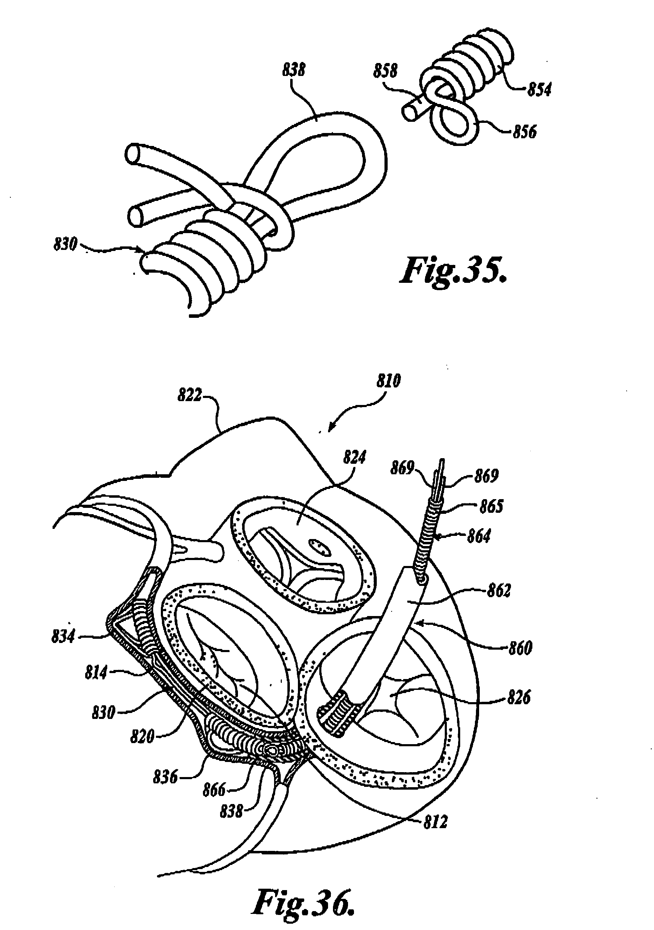

In deploying the device 830, the catheter 852 is first fed into the coronary sinus 814 adjacent the mitral valve annulus 820. The device 830 and pusher 854 are then releasably locked together as shown in FIGURE 34. The device is then loaded into the catheter 852. The pusher 854 follows the device into the catheter 852 and is then advanced along the catheter to push the device 830 distally down the catheter to a predetermined position adjacent the mitral valve annulus 814 at the distal end of the catheter 852. Thereafter, the device is maintained in a stationary position by the pusher 854 as the catheter 852 is partially withdrawn to expose the distal anchor 834. Once the distal anchor 834 is exposed, it is deployed in a manner as fully described in the aforementioned copending Application Serial No. 10/142,637. Once the distal anchor 834 is deployed, the catheter 850 is then retracted proximally of the proximal anchor 836. This exposes the proximal anchor 836. Once the proximal anchor is exposed, the pusher 854 is pulled proximally for tightening the device within the coronary sinus and to an extent which results in the desired effect on the geometry of the mitral valve annulus 820. During this adjustment process, mitral regurgitation may be monitored and the device adjusted for optimal results. When the device 830 is in its final position within the coronary sinus 814, the proximal anchor 836 may then be deployed. The beneficial effect of the device may now again be evaluated. Once the device is ready for chronic implant, the locking pin 858 may be pulled proximally from the proximal end of the pusher 854 as shown in FIGURE 35 to disengage the coupling members 838 and 856. With the pusher 854 now free from the device 830, the pusher 854, catheter 852, coupling member 856 and locking pin 858 may then be removed from the heart.

As can be appreciated by those skilled in the art, guide members, other than a guide catheter as shown herein, may be used to direct the device into the coronary sinus. For example, a guide wire, of the type well known in the art may alternatively be employed to guide the device there along into the coronary sinus without departing from the present invention.

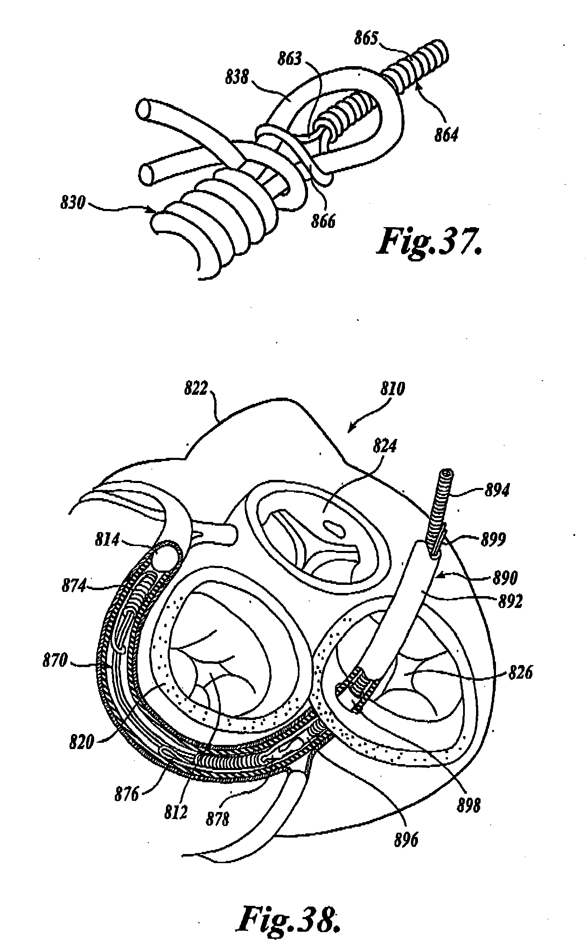

FIGURES 36 and 37 illustrate the manner in which the device 830 may be removed from the coronary sinus 814 if necessary in accordance with further aspects of the present invention. As may be seen in FIGURES 36 and 37, the device 830 may be removed from the coronary sinus 814 with a retractor assembly 860. The retractor assembly includes the catheter 862, and a retractor 864 comprising an elongated coil 865 and a coupling member 866. The elongated coil 865 of the retractor 864 is essentially identical to the pusher 854 as illustrated in FIGURES 33-35. The coupling member 866 may be a cable which extends down the center lumen of the elongated coil 865 to form a loop structure 866 and which then returns through the center lumen of the elongated coil 865 such that the free ends 869 of the cable 863 extend out the proximal end of the elongated coil 865. As also seen in FIGURES 36 and 37, if the device 830 is to be removed from the coronary sinus 814, the cable 863 is threaded into the elongated coil 865 to form the loop structure 866. With the retractor 864 thus formed, the retractor is then guided down the catheter 862 to the proximal end of the device 830 and more specifically to the coupling loop member 838 of the device 830. The loop 866 of the cable 863 is then wrapped about the loop coupling member 838 of the device 830 and the free ends 869 of the cable are drawn proximally to tighten the loop structure 866 about the loop coupling member 838. The retractor 864 now has a grip on the device 830. With the device 830 now being firmly held by the retractor 864, the retractor 864 may be pulled proximally within the catheter 862 to impart proximal movement to the device 830. When the anchors 834 and 836 of the device 830 engage the distal end of the catheter 862, they will be collapsed to disengage from the coronary sinus. The device may now be removed by pulling on the retractor 864 proximally within the catheter 862 until the device is fully removed from the heart and the patient. Alternatively, the device may be drawn into the catheter. The catheter and the device may then be withdrawn together from the patient.

FIGURES 38-40 illustrate a further embodiment of the present invention for releasably locking a pusher member to a mitral valve therapy device for implanting the mitral valve therapy device adjacent the mitral valve annulus within the coronary sinus of the heart.

As illustrated in FIGURE 38, the mitral valve therapy device 870 is elongated and includes a distal anchor 874 and a proximal anchor 876. The anchors are not yet deployed. The device 870 further includes, at its proximal end, a coupling structure 878.

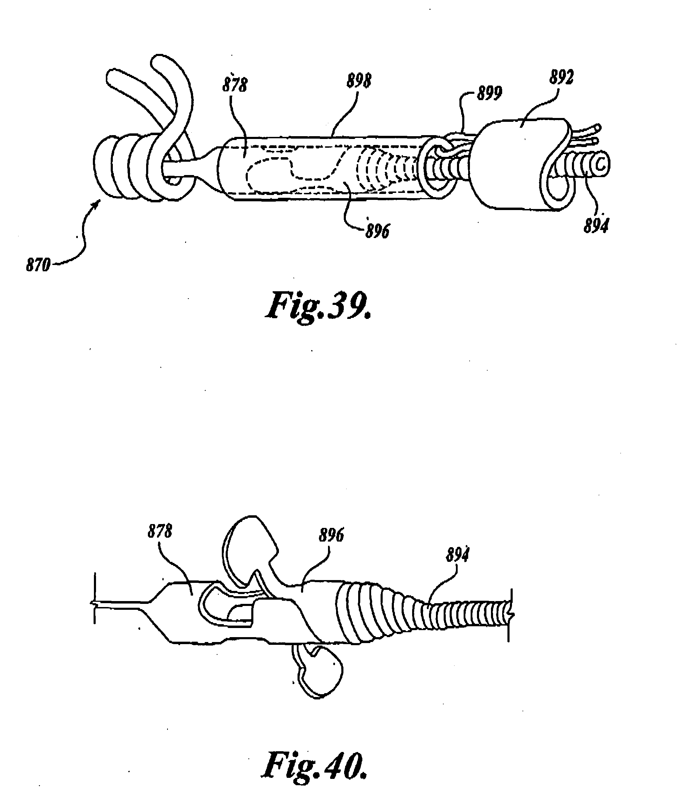

For deploying the device 870, a deployment system 890 is also illustrated. The deployment system includes a catheter 892, a pusher member 894, a coupling structure 896 at the distal end of the pusher 894, and a locking member 898. As will be best seen in FIGURE 39, the coupling member 878 of the device 870 and the coupling member 896 of the pusher 894 form a pair of interlocking structures. The coupling structures 878 and 896 are tubular and the locking member 898 is also tubular.

When it is desired to implant the device 870, the device 870 is coupled to the pusher 898 by the interlocking structures of the coupling members 878 and 896 which are held together and in place by the locking member 898. Then, as previously described in the previous embodiment, the device and pusher member are fed down the catheter 892 until the device reaches a desired position within the coronary sinus adjacent the mitral valve annulus 820. Once in this position, the device is held stationary by the pusher member 894 while the catheter 892 is retracted to expose the distal anchor 874. The distal anchor 874 may now be deployed in a manner as described in the aforementioned copending Application Serial No. 10/142,637. With the distal anchor 874 deployed, the catheter 892 is then retracted until it is proximal to the proximal anchor 876. The pusher 894 may then be pulled to tighten the device within the coronary sinus. Once the device 870 has been tightened to a desired degree, as confirmed by device effectiveness evaluation, the device 870 is ready for chronic implant.

When the device 870 is to be left within the coronary sinus 814, the tether 899 is pulled to slide the locking member 898 off of the interlocking structures 878 and 896. The coupling structures of the pusher 894 may be prestressed for disengaging the coupling structure 878 of the device 870 when the locking member 898 is pulled proximal to the interlocking structures. The device 870 is now free from the pusher member 894. The pusher member 894 together with the tether, locking member, and catheter 892 may be removed from the heart. With the implant of the device 870 completed, the device 870 is left within the coronary sinus adjacent the mitral valve annulus 820 to treat the mitral valve such as by eliminating mitral regurgitation.

As illustrated in FIGURE 40, the coupling structure 896 is prestressed to deflect outwardly when the tubular locking member 898 is pulled proximally to disengage the device 870 from the pusher 894. Alternatively, the coupling structure 896 may be prestressed inwardly with a locking pin (not shown) extending into coupling stricture 878 to maintain the locked arrangement. Here, proximal pulling of the pin would cause the coupling structure 896 to deflect inwardly to disengage the coupling structure 878 and 896.

While the preferred embodiment of the invention has been illustrated and described, it will be appreciated that various changes can be made therein without departing from the scope of the invention. Therefore, the scope of the invention is to be determined from the following claims and equivalents thereto.

### Summaries

1. A device that affects the condition of a mitral valve annulus of a heart comprising:
   an elongated body dimensioned to be placed in the coronary sinus of the heart adjacent the mitral valve annulus;
   a fixation member carried by the device, the fixation member being adjustable from a first configuration that permits placement of the device in the coronary sinus to a second configuration that anchors the device within the coronary sinus; and
   a lock that locks the fixation member in the second configuration.
2. The device of Summary 1 wherein the lock is releasable to release the fixation member from the second configuration to permit the device to be moved within the coronary sinus.
3. The device of Summary 1 wherein the fixation member is deformable to permit the device to be moved within the coronary sinus.
4. The device of Summary 1 wherein the fixation member is adjustable from the first configuration to a maximum second configuration and wherein the lock locks the fixation member intermediate the first configuration and the maximum second configuration.
5. The device of Summary 4 wherein the lock locks the fixation member at any one of a plurality of intermediate points between the first configuration and the maximum second configuration.
6. The device of Summary 1 wherein the fixation member is elongated and has a first end hingedly coupled to the device body, wherein the fixation member extends along the device body closely spaced to the device body when in the first configuration and wherein the fixation member is pivoted from the device body when in the second configuration to engage the coronary sinus and anchor the device in the coronary sinus.
7. The device of Summary 6 further comprising a support that renders the fixation member substantially rigid when in the second configuration.
8. The device of Summary 7 wherein the support is an extension of the fixation member, wherein the fixation member includes a second end opposite the first end and wherein the lock locks the fixation member second end on the device body.
9. The device of Summary 8 wherein the fixation member second end is slidable along the device body.
10. The device of Summary 8 wherein the device comprises a plurality of the fixation members.
11. The device of Summary 7 wherein the fixation member includes a second end opposite the first end, wherein the support comprises a support member having a first end hingedly coupled to the fixation member second end, wherein the support member has a second end opposite the support member first end, and wherein the lock locks the support member second end on the device body.
12. The device of Summary 11 wherein the support member second end is slidable along the device body.
13. The device of Summary 11 wherein the device comprises a plurality of the fixation members and support members.
14. An assembly that affects the condition of a mitral valve annulus of a heart, the assembly comprising:
   a mitral valve therapy device dimensioned to be placed in the coronary sinus adjacent the mitral valve annulus, the device including an elongated body, a fixation member carried by the device, the fixation member being adjustable from a first configuration that permits placement of the device in the coronary sinus to a second configuration that anchors the device within the coronary sinus, and a lock that locks the fixation member in the second configuration; and
   a flexible catheter having a lumen that receives the device and being dimensioned to be advanced into the coronary sinus to place the device adjacent the coronary sinus.
15. The assembly of Summary 14 wherein the fixation member is adjustable from the first configuration to a maximum second configuration and wherein the lock locks the fixation member intermediate the first configuration and the maximum second configuration.
16. The assembly of Summary 15 wherein the lock locks the fixation member at any one of a plurality of intermediate points between the first configuration and the maximum second configuration.
17. The assembly of Summary 14 further comprising an elongated pusher that is received by the lumen of the catheter proximal to the device and that permits the device and the catheter to be moved opposite each other.
18. The assembly of Summary 14 further comprising a tether receivable by the catheter lumen and engagable with the device to pull the device distally with respect to the catheter.
19. The assembly of Summary 18 wherein the catheter transitions the fixation member from the first configuration to the second configuration.
20. The assembly of Summary 19 wherein the fixation member is elongated and has a first end hingedly coupled to the device body, wherein the fixation member extends along the device body when in the first configuration and wherein the fixation member is pivoted from the device body into the second configuration by distal movement of the catheter with respect to the device to engage the coronary sinus and anchor the device in the coronary sinus.
21. The assembly of Summary 20 wherein the device further comprises a support that renders the fixation member substantially rigid when in the second configuration.
22. The assembly of Summary 21 wherein the support is an extension of the fixation member, wherein the fixation member includes a second end opposite the first end and wherein the lock locks the fixation member second end on the device body when the fixation member second end is in a locked position.
23. The assembly of Summary 22 wherein the fixation member second end is slidable along the device body by the catheter into the locked position.
24. The assembly of Summary 22 wherein the device comprises a plurality of the fixation members.
25. The assembly of Summary 21 wherein the fixation member includes a second end opposite the first end, wherein the support comprises a support member having a first end hingedly coupled to the fixation member second end, wherein the support member has a second end opposite the support member first end, and wherein the lock locks the support member second end on the device body when the support member second end is in a locked position.
26. The assembly of Summary 25 wherein the support member second end is slidable along the device body by the catheter into the locked position.
27. The assembly of Summary 26 wherein the device comprises a plurality of the fixation members and support members.
28. The assembly of Summary 14 wherein the lock is releasable to release the fixation member from the second configuration to permit the device to be removed from the coronary sinus.
29. The assembly of Summary 14 wherein the fixation member is deformable to permit the device to be moved within the coronary sinus.
30. An anchor that anchors, in a body lumen, a device having an elongated body dimensioned to be placed in the body lumen, the anchor comprising:
   a fixation member carried on the device, the fixation member being adjustable from a first configuration that permits placement of the device in the body lumen to a second configuration that anchors the device within the body lumen; and
   a lock that locks the fixation member in the second configuration.
31. The anchor of Summary 30 wherein the lock is releasable to release the fixation member from the second configuration to permit the device to be moved within the body lumen.
32. The anchor of Summary 30 wherein the fixation member is deformable to permit the device to be moved within the body lumen.
33. The anchor of Summary 30 wherein the fixation member is adjustable from the first configuration to a maximum second configuration and wherein the lock locks the fixation member intermediate the first configuration and the maximum second configuration.
34. The anchor of Summary 33 wherein the lock locks the fixation member at any one of a plurality of intermediate points between the first configuration and the maximum second configuration.
35. The anchor of Summary 30 wherein the fixation member is elongated and has a first end hingedly coupled to the device body, wherein the fixation member extends along the device body closely spaced to the device body when in the first configuration and wherein the fixation member is pivoted from the device body when in the second configuration to engage the body lumen and anchor the device in the body lumen.
36. The anchor of Summary 35 further comprising a support that renders the fixation member substantially rigid when in the second configuration.
37. The anchor of Summary 36 wherein the support is an extension of the fixation member, wherein the fixation member includes a second end opposite the first end and wherein the lock locks the fixation member second end on the device body.
38. The anchor of Summary 37 wherein the fixation member second end is slidable along the device body.
39. The anchor of Summary 37 comprising a plurality of the fixation members.
40. The anchor of Summary 36 wherein the fixation member includes a second end opposite the first end, wherein the support comprises a support member having a first end hingedly coupled to the fixation member second end, wherein the support member has a second end opposite the support member first end, and wherein the lock locks the support member second end on the device body.
41. The anchor of Summary 40 wherein the support member second end is slidable along the device body.
42. The anchor of Summary 40 comprising a plurality of the fixation members and support members.
43. A device for modifying the shape of tissue adjacent to a lumen, the device comprising :
   an anchor adapted to securely contact a lumen wall when the device is deployed in a lumen, the anchor having an unexpanded delivery configuration and an expanded deployed configuration;
   a lock locking the anchor in its expanded deployed configuration; and
   a reshaper extending from the anchor and adapted to contact the lumen wall when the device is deployed in the lumen,
   the anchor comprising a flexible member extending from the reshaper in a curved path not parallel to the central axis of the lumen.
44. The device of Summary 43 wherein the lock comprises a locking element connected to the flexible member.
45. The device of Summary 43 wherein the flexible member is adapted to extend from the axis of the reshaper in at least a first place and a second place.
46. The device of Summary 45 wherein the flexible member comprises two arms extending from the first place and two arms extending from the second place.
47. The device of Summary 46 wherein the flexible member is adapted to have the two arms extending from the first place and the two arms extending from the second place meet at a third place.
48. The device of Summary 47 wherein the flexible member is adapted to have the third place outside of the axis of the reshaper.
49. The device of Summary 48 wherein the flexible member is formed in a bowed figure eight pattern.
50. The device of Summary 46 wherein the lock comprises a locking element connected to the flexible member.
51. The device of Summary 50 wherein the locking element comprises a loop connecting the two arms extending from the first place.
52. The device of Summary 51 wherein the locking element comprises a first locking element, the lock further comprising a second locking element connected to the reshaper.
53. The device of Summary 52 wherein the reshaper comprises a support wire, the second locking element comprising a bend in the support wire.
54. The device of Summary 52 wherein the second locking element comprises a camming surface extending from the reshaper.
55. The device of Summary 43 wherein the lock comprises a first locking element connected to the flexible member, the lock further comprising a second locking element connected to the reshaper.
56. The device of Summary 55 wherein the reshaper comprises a support wire, the second locking element comprising a bend in the support wire.
57. The device of Summary 55 wherein the second locking element comprises a camming surface extending from the reshaper.
58. The device of Summary 43 further comprising a crimp, the flexible member comprising two ends held by the crimp.
59. The device of Summary 58 wherein the flexible member is adapted to extend from an axis of the crimp in at least a first place and a second place, the first place comprising one end of the crimp.
60. The device of Summary 59 wherein the second place comprises another end of the crimp.
61. An assembly for affecting the condition of a mitral valve annulus of a heart comprising:
   a mitral valve therapy device that reshapes the mitral valve annulus of the heart when placed within the coronary sinus of the heart adjacent the mitral valve annulus, the mitral valve therapy device having a proximal end including a coupling structure;
   a catheter having a lumen that directs the mitral valve therapy device into the coronary sinus of the heart;
   a second coupling structure that is lockable on the device coupling structure; and
   a locking member that locks the device coupling structure to the second coupling structure and that releases the device coupling structure from the second coupling structure.
62. The assembly of Summary 61 further including a pusher member that pushes the device through the catheter lumen, the pusher member having a distal end that engages the device proximal end.
63. The assembly of Summary 62 wherein the pusher member carries the second coupling structure at the distal end of the pusher member.
64. The assembly of Summary 61 wherein the device coupling structure comprises a hoop structure.
65. The assembly of Summary 64 wherein the second coupling structure comprises a hoop structure.
66. The assembly of Summary 65 wherein the locking member comprises a pin that extends through the hoop structures to lock the coupling structures together and that is retractable to release the hoop structures.
67. The assembly of Summary 66 wherein the catheter has a distal end and
   wherein the pin is elongated and extends through the distal end of the catheter.
68. The assembly of Summary 66 further comprising a pusher member having a lumen and that pushes the device through the catheter lumen, the pusher member having a distal end that engages the device proximal end, a proximal end, and a lumen, and wherein the pin is elongated and extends through the pusher member lumen and out the pusher member proximal end.
69. The assembly of Summary 68 wherein the pusher member is an elongated coil.
70. The assembly of Summary 61 wherein the device coupling structure and the second coupling structure comprise a pair of interlocking structures and wherein the locking member comprises a slide-lock sheath closely fitted to the interlocking structures.
71. The assembly of Summary 70 wherein the interlocking structures are formed from tubing and wherein the slide-lock sheath is tubular.
72. The assembly of Summary 70 further including a pusher member that pushes the device through the catheter lumen, the pusher member having a distal end that engages the device proximal end, and wherein the pusher member carries the second coupling structure at the distal end of the pusher member.
73. The assembly of Summary 72 wherein the locking member further comprises a tether that extends from the slide-lock sheath to and through the catheter lumen to permit the tether to pull proximally on the slide-lock sheath for releasing the interlocking structures.
74. The assembly of Summary 61 further comprising a retractor configured to extend through the catheter lumen and grip the device coupler to permit retraction of the device through the catheter.
75. The assembly of Summary 74 further comprising a pusher member configured to extend through the catheter lumen and that pushes the device through the catheter lumen, the pusher member having a lumen and a distal end adapted to engage the device proximal end, and wherein the retractor is configured to extend down the pusher lumen to engage the device coupler.
76. An assembly for affecting the condition of a mitral valve annulus of a heart comprising:
   device means for reshaping the mitral valve annulus of the heart when placed within the coronary sinus of the heart adjacent the mitral valve annulus, the device means having a proximal end including a coupling means for coupling the device means;
   catheter means having a lumen that directs the mitral valve therapy device into the coronary sinus of the heart;
   second coupling means for locking with the device coupling means; and
   locking means for locking the device coupling means to the second coupling means and releasing the device coupling means from the second coupling means.
77. The assembly of Summary 76 further comprising a pusher means for pushing the device means through the catheter means lumen, the pusher means having a lumen, and wherein the second coupling means and the locking means extend through the pusher means lumen.
78. The assembly of Summary 76 wherein the device coupling means and the second coupling means comprise interlocking means for releasably locking the device coupling means and the second coupling means, and wherein the locking means includes retaining means for retaining the interlocking means in an interlocked condition.
79. The assembly of Summary 78 wherein the retaining means is displaceable for releasing the interlocked condition of the interlocking means.
80. The assembly of Summary 76 further comprising retracting means for engaging the device coupling means and removing the device means from the coronary sinus.
81. An assembly for effecting the condition of a mitral valve annulus of a heart comprising;
   a mitral valve therapy device that reshapes the mitral valve annulus of the heart when placed within the coronary sinus of the heart adjacent the mitral valve annulus, the mitral valve therapy device having a proximal end including a coupling structure;
   a guide member that directs the mitral valve therapy device into the coronary sinus of the heart;
   a second coupling structure that is lockable on the device coupling structure; and
   a locking member that locks the device coupling structure to the second coupling structure and that releases the device coupling structure from the second coupling structure.
82. An assembly for effecting the condition of a mitral valve annulus of a heart comprising:
   device means for reshaping the mitral valve annulus of the heart when placed within the coronary sinus of the heart adjacent the mitral valve annulus, the device means having a proximal end including a coupling means for coupling the device means;
   guide means for directing the mitral valve therapy device into the coronary sinus of the heart;
   second coupling means for locking with the device coupling means; and
   locking means for locking the device coupling means to the second coupling means and releasing the device coupling means from the second coupling means.
83. A device for modifying the shape of tissue adjacent to a lumen, the device comprising:
   an anchor adapted to be deployed by a catheter to engage the lumen;
   a support extending from the anchor and adapted to engage the lumen; and
   a recapture mechanism adapted to assist the recapture of at least a portion of the device into the catheter.
84. The device of Summary 83 wherein the anchor comprises a proximal portion, the recapture mechanism comprising a surface on the proximal portion of the anchor adapted to guide the catheter over the device.
85. The device of Summary 84 wherein anchor is further adapted to expand after deployment from the catheter.
86. The device of Summary 85 wherein the recapture mechanism is adapted to assist in compression of the anchor.
87. The device of Summary 85 wherein the recapture mechanism comprises a first surface on the anchor and a second surface on the anchor, the slope of first surface with respect to a longitudinal axis of the anchor being less than the slope of the second surface with respect to the longitudinal axis of the anchor.
88. An intravascular support comprising:
   a proximal anchor that circumferentially engages a vessel wall in which the support is placed, the proximal anchor being disposed in a proximal anchor plane;
   a distal anchor that circumferentially engages a vessel wall in which the support is placed, the distal anchor being offset in a distal anchor plane offset from the proximal anchor plane; and
   a support wire extending between the proximal and distal anchors so that the support wire is urged against the vessel wall to compress tissue that is adjacent the vessel.
89. The intravascular support of Summary 88 wherein the proximal anchor and the distal anchor each comprise a wire and a crimp connected to the wire.
90. The intravascular support of Summary 89 wherein the proximal anchor and the distal anchor each comprise a plurality of wires.
91. The intravascular support of Summary 90 wherein the crimp comprises a bore, the bore of the crimp having a non-circular shape adapted to accommodate and orient the wires.
92. The intravascular support of Summary 91 wherein the bore is substantially square.

## Claims

1. A device for treating mitral valve regurgitation, the device comprising:
(i) a proximal anchor and distal anchor;
at least one of the distal anchor and the proximal anchor being collapsible and expandable between a first configuration and a second configuration;
wherein the first configuration is a collapsed configuration which occurs during delivery or recapture of the device and the second configuration is an expanded configuration for when disposed within a cardiac vein anchoring the device;
(ii) an elongate support member extending from the distal anchor which can apply sufficient pressure on the cardiac vein wall to modify tissue adjacent the support member when the device is anchored; and
(iii) said device being adapted for delivery and at least partial recapture of at lease one of the distal anchor and the proximal anchor to and from the cardiac vein through a catheter;
(iv) so that the distal anchor and the proximal anchor may be delivered through a catheter, following delivery at least one of the distal anchor and the proximal anchor expands thereby anchoring the device, and thereafter during recapture into the catheter at least one of the distal anchor and the proximal anchor collapses to permit such recapture.

2. A device for treating mitral valve regurgitation as claimed in claim 1 wherein the distal anchor is collapsible and expandable, said device is adapted for delivery and recapture of the distal anchor, so that the distal anchor expands anchoring the device and during recapture collapses to permit recapture.

3. A device for treating mitral valve regurgitation as claimed in either of claims 1 and 2 wherein adapted such that distally directed force from the delivery catheter collapses the collapsible anchor.

4. A device for treating mitral valve regurgitation as claimed in any of claims 1 to 3 which further comprises a lock, the expandable anchor in the expanded configuration.

5. A device for treating mitral valve regurgitation as claimed in claim 4 wherein the expandable anchor is adapted to be recaptured subsequent to having been locked in the expanded configuration.

6. A device for treating mitral valve regurgitation as claimed in any of claims 1 to 5 wherein the expandable anchor is adapted to be fully recaptured back within the delivery catheter.

7. A device for treating mitral valve regurgitation as claimed in any of claims 1 to 5 wherein the expandable anchor is adapted to be partially recaptured back within the delivery catheter.

8. A device for treating mitral valve regurgitation as claimed in any of claims 1 to 7 wherein both proximal and distal anchors are expandable.

9. A device for treating mitral valve regurgitation as claimed in claim 8 where both proximal and distal anchors are adapted to be recaptured back within the delivery catheter in a collapsed configuration.

10. The device for treating mitral valve regurgitation as claimed in claim 1 wherein an expandable anchor or anchors comprise a recapture assist element.
